# EUROPEAN PATENT SPECIFICATION

(11) **EP 2 216 349 B1**
(45) Date of publication and mention of the grant of the patent: **20.09.2023**
(21) Application number: 08853755.0
(22) Date of filing: 26.11.2008
(51) Int. Cl.: C08F 4/68, A61K 6/30, A61K 6/887

(54) **USE OF A PHENOL TYPE COMPOUND IN A DENTAL CHEMICAL POLYMERIZATION CATALYST WITH IMPROVED POLYMERIZING ACTIVITY**
VERWENDUNG EINER VERBINDUNG DES PHENOLTYPS IN EINEM DENTAL-CHEMISCHEN POLYMERISATIONSKATALYSATOR MIT VERBESSERTER POLYMERISATIONSAKTIVITÄT
UTILISATION D'UN COMPOSÉ DE TYPE PHENOL DANS UN CATALYSEUR DE POLYMÉRISATION CHIMIQUE DENTAIRE A ACTIVITÉ DE POLYMÉRISATION AMELIORÉE

(30) Priority: 27.11.2007 JP 2007305858
(43) Date of publication of application: 11.08.2010
(73) Proprietor: Tokuyama Dental Corporation, Tokyo 110-0016 (JP)
(72) Inventor: KIMURA, Mikio, Tokyo 110-0016 (JP); KAKIUCHI, Naoki, Tokyo 110-0016 (JP)
(74) Representative: J A Kemp LLP
(86) International application number: PCT/JP2008/071447
(87) International publication number: WO 2009/069657

(56) References cited:
- JP-A- 2003 096 122
- JP-A- 2004 203 773
- JP-A- 2004 284 969
- JP-A- 2005 239 560

## Description

### Technical Field:

This invention relates to a chemical polymerization catalyst used in the field of dental therapy and to a dental curable composition blended with the catalyst.

### Background Art:

A method of curing a polymerizable monomer by using a polymerization catalyst has been widely used in the dental field, and examples of the curable composition that utilizes this method include dental cement, dental adhesive, composite resin, dental self curing resin, dental pre-treating material and the like.

For these curable composition, a polymerization catalyst is selectively used depending upon their compositions, objects of use and required properties. The polymerization catalysts include photopolymerization catalysts and chemical polymerization catalysts. A chemical polymerization catalyst must be used in case when the curable composition contains much filler that little permits the transmission of light or when the curable composition is used for such an application where it is difficult to apply light. Further, the dental adhesive and the pre-treating material must often use a chemical polymerization catalyst depending upon the kind of the dental material to which the adhesion is to be made. When the photopolymerization catalyst is used, furthermore, it becomes necessary to apply light by using a dedicated device and, therefore, it is often desired to use a chemical polymerization catalyst from the standpoint of simplifying the operation.

The photopolymerization catalyst which utilizes light energy is highly active enabling the curing by polymerization to quickly proceed whereas the chemical polymerization catalyst which does not utilize external energy such as light is accompanied by a problem of low activity. In recent years, however, various chemical polymerization catalysts have been proposed featuring improved activity. For instance, a patent document 1 proposes a chemical polymerization catalyst comprising an aryl borate compound and an acid compound, and a patent document 2 discloses a chemical polymerization catalyst obtained by adding an organic oxide and/or a vanadium compound having a valency of +IV to +V to the system comprising an aryl borate compound and an acid compound.

In the chemical polymerization catalysts proposed by the patent documents 1 and 2, it is considered that the aryl borate compound reacts with the acid compound to form a triphenylborane as represented by the following formula, and benzo radicals generated from the formed triphenylborane serve as an initiator to accelerate the polymerization reaction.

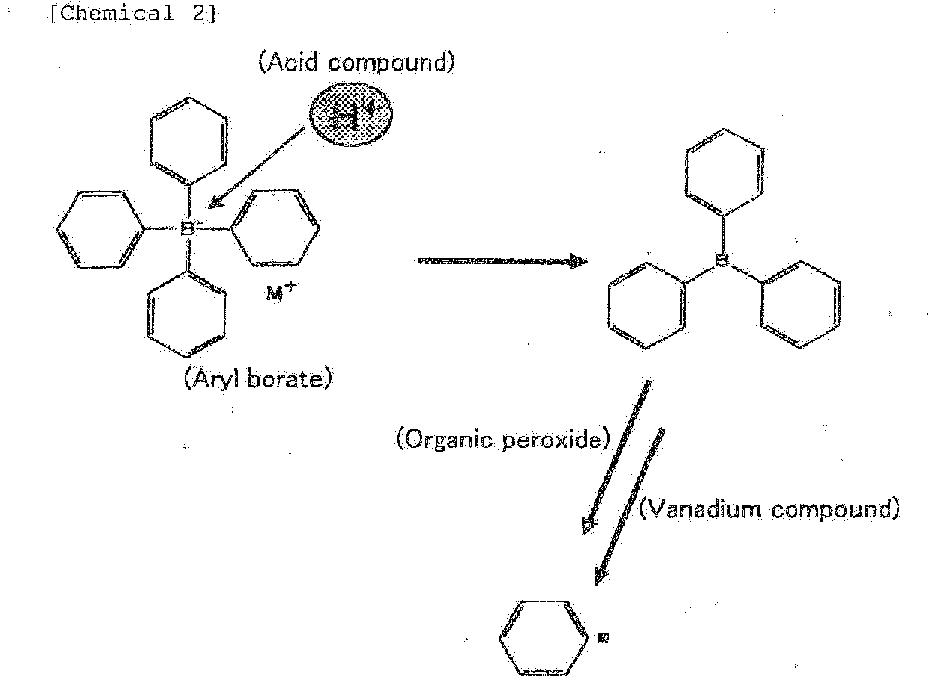

That is, as will be understood from the above formula, upon being blended with the organic peroxide and the vanadium compound having a valency of +IV to +V, the chemical polymerization catalyst of the patent document 2 accelerates the formation of benzo radicals from the arylborane such as triphenylborane to thereby, further, enhance the polymerizing activity of the chemical polymerization catalyst disclosed in the patent document 1. Patent document 3 discloses a curable composition wherein hydroquinone, hydroquinone monomethyl ether and 2,5-di-t-butyl cresol are used as polymerization inhibitors.
Patent document 1: JP-A-9-227325
Patent document 2: JP-A-2002-187907
Patent document 3: JP-A-2004-203773

### Disclosure of the Invention:

### Problems that the Invention is to Solve:

However, none of the above - chemical polymerization catalysts, i.e., neither the chemical polymerization catalyst- of the patent document 1 nor the chemical polymerization catalyst of the patent document 2 exhibit polymerizing activity sufficient for use in the field of dental therapy where the polymerization and curing are in many cases carried out in the oral cavity. Therefore, it has been desired to provide a chemical polymerization catalyst which exhibits further increased polymerizing activity.

With the above chemical polymerization catalysts, further, improved polymerizing activity can be obtained by increasing, for example, the amount of the vanadium compound having a valency of +IV to +V accompanied, however, by a great decrease in the storage stability. That is, the above chemical polymerization catalysts are, usually, stored in a manner that a component containing the aryl borate compound and the polymerizable monomer is stored in one package, and a component - containing the acid compound, the vanadium compound and the polymerizable monomer is stored in another package. At the time of use, the two components are mixed together to prepare a paste. The paste is then applied to a predetermined part or is adjusted into a predetermined shape, and is polymerized and cured. When put to use by mixing them together after stored for extended periods of time, however, the paste is often gelled making it difficult to apply the paste to the predetermined part or to adjust the paste into the predetermined shape impairing the practicability, which is a fundamental problem. Further, an increase in the amount of the vanadium compound brings about such an inconvenience as imparting a ,color to the cured body. Therefore, it cannot be expected to improve the polymerizing activity by increasing the amount of the vanadium compound, and it has been urged to improve the polymerizing activity by any other means.

It is, therefore, an object of the present invention to provide a dental chemical polymerization catalyst comprising, particularly, a combination of an aryl borate compound and an acid compound, and exhibiting not only excellent polymerizing activity but also storage stability.
Another object of the present invention is to provide a chemical polymerization catalyst which also effectively suppresses the problem of imparting a color to the cured body.
A further object of the present invention is to provide a dental curable composition which contains the above chemical polymerization catalyst, and can be applied to a variety of uses such as adhesive material, primer, composite resin, prosthetic and the like.

### Means for Solving the Problem:

The present inventors have conducted a keen study concerning a chemical polymerization catalyst system that uses an aryl borate compound and an acid compound in combination, have newly discovered the fact that when a phenol type compound having a chemical structure suppressing steric hindrance is used together with a vanadium compound having a valency of +IV to +V, the polymerizing activity can be markedly improved without impairing storage stability, requiring the vanadium compound in small amounts and without imparting color to the cured body, and have thus completed the invention.

Namely, according to the present invention, there is provided the use of a phenol type compound represented by the following general formula (1), 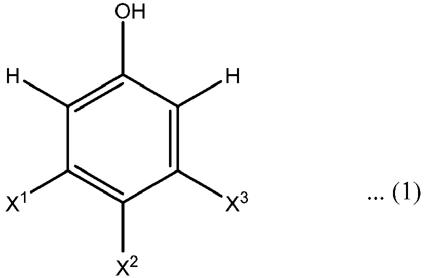
wherein X¹, X² and X³ are each a hydrogen atom or a substituent selected from the group consisting of a hydroxyl group, an alkoxy group and an alkyl group;
   for improving the polymerizing activity of a dental chemical polymerization catalyst, wherein the use is not in a method of treatment of the human or animal body by surgery or therapy, and wherein the dental chemical polymerization catalyst comprises:
   (A) an aryl borate compound;
   (B) an acid compound which is an acid group-containing polymerizable monomer;
   (C) a vanadium compound having a valency of +IV or +V; and
   (D) the phenol type compound represented by general formula (1);
said acid compound (B) being contained in an amount of 0.1 to 100 mols per mole of the aryl borate compound (A) and said vanadium compound (C) being contained in an amount of 0.0005 to 0.015 mol per mol of said aryl borate compound (A), and wherein said phenol type compound (D) is contained in an amount of 0.05 to 200 mols per mol of said vanadium compound (C) and said phenol type compound (D) is not the compound in which X1 and X3 are hydrogen atoms and X2 is a methoxy group.

In the dental chemical polymerization catalyst, it is desired that:
(1) An organic peroxide (E) is, further, contained;
(2) A compound containing not less than two phenolic hydroxyl groups is used as the phenol type compound (D);
(3) A hydroquinone is used as the phenol type compound (D); and
(4) The aryl borate compound (A) is stored in a form of package separate from the acid compound (B), vanadium compound (C) and phenol type compound (D), and the components (A) to (D) are all mixed together at the time of use.

### Effects of the Invention:

The invention has an important feature in that the phenol type compound (component D) represented by the above) represented by the above general formula (1) together with the vanadium compound (component C) are used in combination with the aryl borate compound (component A) and the acid compound (component B) which are the fundamental components. Owing to this composition, the chemical polymerization catalyst exhibits excellent polymerizing activity even without being irradiated with light such as ultraviolet rays. Therefore, the composition that contains the catalyst components (A) to (D) as well as a polymerizable monomer component undergoes the polymerization and curing within short periods of time to form a cured body without fluidity. As demonstrated in Comparative Example 1 appearing later, for example, a composition containing the catalyst components (A) to (C) and the polymerizable monomer but without containing the above phenol type compound requires a curing time (initial curing time) immediately after the preparation of the composition until the formation of the cured body without fluidity of 300 seconds whereas the composition which, further, contains the above phenol type compound (component D) of the present invention requires the initial curing time which is as markedly shortened as 140 seconds to 260 seconds (Examples 1 to 24) exhibiting greatly improved polymerizing activity.

Further, the invention requires the vanadium compound (component C) of a valency of +IV to +V in only a very small amount to secure the polymerizing activity and, therefore, features a favorable storage stability while effectively avoiding such an inconvenience that the cured body is colored.

For example, the aryl borate compound (component A) and the acid compound (component B) for constituting the catalyst are stored a form of separate packages each, however, containing a liquid polymerizable monomer component. That is, if both of the above components (A) and (B) are made present together in combination with the polymerizable monomer, then the polymerization takes place. In this case, in order to avoid the start of polymerization, too, the vanadium compound (component C) having a valency of +IV to +V is stored separately from the aryl borate compound (component A) but being mixed with the acid compound (component B).

In the storage stability test of Example 1 appearing later, there were prepared a composition A1 by adding the vanadium compound (component C) and the phenol type compound (component D) to the polymerizable monomer, and a composition B1 by adding the polymerizable monomer to the aryl borate compound (component A), these compositions were stored at 37°C for one month and were, thereafter, mixed together to obtain a paste thereof of which the curing time was 185 seconds. That is, as compared to the initial curing time (180 seconds) of when the compositions A1 and B1 just after the preparation thereof were mixed together and were polymerized and cured, the above curing time was not almost different manifesting the maintenance of excellent polymerizing activity.

. In Comparative Example 2 appearing later, on the other hand, a BHT (2, 6-di-t-butylhydroxytoluene) which has been known as a polymerization inhibitor was added, instead of the phenol type compound of the general formula (1) used in the present invention, to a composition A2 that contained the acid compound (component B) and the vanadium compound (component C), and the initial curing time and the curing time after stored at 37°C for one month were measured in quite the same manner as in Example 1 but using the composition A2. That is, the initial curing time was 330 seconds while the curing time after stored for one month was 340 seconds. Namely, use of the polymerization inhibitor could suppress a drop in the polymerizing activity caused by the storage for extended periods of time causing, however, the curing time to be very prolonged, from which it was learned that the polymerizing activity had already been lowered from the initial step.

In Comparative Example 3 appearing later, further, a composition A3 containing the acid compound was prepared by using the vanadium compound (component C) in an amount 5 times as great as that of Example 1 together with the BHT but without using the phenol type compound of the general formula (1) used in the present invention, and the initial curing time and the curing time after stored at 37°C for one month were measured in quite the same manner as in Example 1 but using the composition A3. The initial curing time was 180 seconds and the polymerizing activity has very increased to be comparable to that of the present invention. After stored at 37°C for one month, however, the composition itself was gelled and the storage stability has greatly decreased.

As will be understood from the experimental results of the above Example 1 and Comparative Examples 1 to 3, when the phenol - type compound (component D) of the general formula (1) is used together with the vanadium compound (component C) having a valency of +IV to +V according to the present invention, it is made possible to greatly improve the polymerizing activity while maintaining storage stability using the vanadium compound not in so large amounts. On the other hand, when use is made of the phenol type compound such as BHT which has been used as a polymerization inhibitor but which is different from the component represented by the general formula (1), the storage stability can be secured but the polymerizing activity cannot be enhanced. In order to enhance the polymerizing activity, further, it becomes necessary to use the vanadium compound in large amounts which spoils the storage stability.

Though the reason has not yet been clarified why, according to the invention, use of the phenol type compound (component D) of the general formula (1) brings- about a marked improvement in the polymerizing activity without impairing the storage stability, the inventors presume that the phenol type compound has no substituent at the second or sixth position, has a low degree of steric hindrance, has a highly reactive OH group in the molecules thereof and, therefore, exchanges ligands of the vanadium compound having a valency of +IV to +V to enhance its activity contributing to selectively forming benzo radicals from the arylborane such as triphenylborane without trapping the formed radicals. Namely, it is considered that the phenol type compound such as BHT has a high degree of steric hindrance causing OH groups to exhibit_low activity and, therefore, does not contribute to enhancing the activity by the exchange of ligands of the vanadium compound having a valency of +IV to +V, exhibiting merely a function as a polymerization inhibitor for trapping the formed radicals. Therefore, though the storage stability can be improved, the polymerizing activity is not improved.

Further, the chemical polymerization catalyst contains the acid compound (component B) and exhibits an etching effect. When applied to the damaged part of a tooth, therefore, the,curable composition blended with the chemical polymerization catalyst penetrates into the dentin of the tooth offering, therefore, an advantage of improved adhesion to the tooth.

As described above, the chemical polymerization catalyst of the invention has such a high polymerizing activity as to quickly execute the curing in an oral environment even without being irradiated with light and, besides, has excellent storage stability. Therefore, the chemical polymerization catalyst of the invention can be effectively used in the field of dental therapy. Accordingly, the curable composition blended with the chemical polymerization catalyst can be preferably used as a dental restorative like a composition resin for filling a cavity of a damaged tooth, as an adhesive like a dental cement for fixing the dental restorative to the tooth or as a primer for application to a tooth for improving adhesion between the tooth and the dental restorative or the dental cement. It is also allowable to apply the above chemical polymerization catalyst to the use of the dental prosthetics such as onlay, inlay, etc.

### Best Mode for Carrying Out the Invention:

### <Chemical polyimerization catalyst>

The chemical polymerization catalyst is used as a polymerization catalyst for various curable compositions used in the field of dental therapy, contains (A) an aryl borate compound, (B) an acid compound which is an acid group-containing polymerizable monomer, (C) a vanadium compound of a valency of +IV to +V and (D) a phenol type compound as essential components and, further, suitability contains (E) an organic peroxide to further improve the catalytic activity.

The catalyst components will now be described.

### (A) Aryl borate compounds:

The aryl borate compound used for the chemical polymerization catalyst of the invention has at least one boron-aryl bond in the molecules, and is concretely expressed by the following general formula (2), 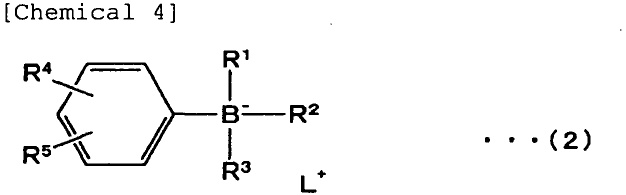
wherein R¹, R² and R³ are each an alkyl group, an aryl group, an aralkyl group or an alkenyl group,
R⁴ and R⁵ are each a halogen atom, an alkyl group or a phenyl group, and
L⁺ is a metal cation, a quaternary ammonium ion, a quaternary pyridinium ion, a quaternary quinolinium ion or a phosphonium ion.

As will be understood from the reaction mechanism of a catalyst system containing the above aryl borate compound and the acid compound, the aryl borate compound serves as a source of radicals for commencing the polymerization. In this case, a borate compound having no boron-aryl bond, too, can serve as a source of radicals, which, however, cannot be used as a source of radicals in the present invention because of its poor storage stability. That is, in the dental clinic, the material that utilizes the curing reaction of the chemical polymerization catalyst system has its catalyst components stored being contained in separate packages and just before the use, the separately packaged components are mixed and kneaded together so as to be used as a paste of a curable composition. Here, however, the borate compound having no boron-aryl bond easily reacts with oxygen in the air and is decomposed. Therefore, even in the packaged state, the borate compound without boron-aryl bond is easily deteriorated or readily undergoes the curing reaction at the time of mixing or kneading failing to provide ample operation time for filling the damaged part of the tooth with the past of the curable composition or for molding the paste of the curable composition into a predetermined shape, making it virtually difficult to use the compound. The aryl borate compound used in the present invention, on the other hand, has a suitable degree of stability and does not bring about the above problems.

In the general formula (2) representing the structure of the aryl borate compound, the groups R¹ to R³ are alkyl groups, aryl groups, aralkyl groups or alkenyl groups, which may have a substituent.

Among these groups, the alkyl group may be of the form of either a straight chain or a branched chain without any particular limitation. Desirably, however, the alkyl group is an alkyl group having 3 to 30 carbon atoms and, more desirably, a straight chain alkyl group having 4 to 20 carbon atoms, such as n-butyl group, n-octyl group, n-dodecyl group and h-hexadecyl group. As the substituent which may be possessed by the alkyl group, there can be exemplified a halogen atom such as fluorine atom, chlorine atom or bromine atom, a hydroxyl group, a nitro group, a cyano group or an aryl group having 6 to 10 carbon atoms, such as phenyl group, nitrophenyl group or chlorophenyl group, an alkoxy group having 1 to 5 carbon atoms, such as methoxy group, ethoxy group or propoxy group, or an acyl group having 2 to 5 carbon atoms, such as acetyl group. There is no particular limitation on the number and positions of the substituents.

There is no particular limitation, either, on the aryl group which, therefore, may have a substituent. Desirably, however, the aryl group has 6 to 14 carbon atoms (excluding those carbon atoms possessed by the substituent) with which a single ring or two or three rings are condensed. As the substituent which may be possessed by the aryl group, there can be exemplified those groups mentioned above as the substituents for the alkyl group, as well as alkyl groups having 1 to 5 carbon atoms, such as methyl group, ethyl group and butyl group.

Concrete examples of the aryl group include:
phenyl group,
1- or 2-naphthyl group,
1-, 2- or 9-anthryl group,
1-, 2-, 3-, 4- or 9-phenanthryl group,
p-fluorophenyl group,
p-chlorophenyl group,
(3,5-bistrifluoromethyl)phenyl group,
3,5-bis(1,1,1,3,3,3-hexafluoro-2-methoxy-2-propyl) phenyl group,
p-nitrophenyl group,
m-nitrophenyl group,
p-butylphenyl group,
m-butylphenyl group,
p-butyloxyphenyl group,
m-butyloxyphenyl group,
p-octyloxyphenyl group,
m-octyloxyphenyl group, etc.

There is no particular limitation on the aralkyl group which, therefore, may, further, have a substituent but, generally, has 7 to 20 carbon atoms (excluding the carbon atoms of the substituent), and may be, for example, benzyl group, phenetyl group or tolyl group. As the substituent, there can be exemplified those mentioned above for the aryl group.

There is no particular limitation, either, on the alkenyl group which, therefore, may have a substituent and is, preferably, an alkenyl group having 4 to 20 carbon atoms (excluding the carbon atoms of the substituent), such as 3-hexenyl group or 7-octenyl group. As the substituent, there can be exemplified those mentioned above as the substituents for the alkyl group.

In the above general formula (1), R⁴ and R⁵ are each a hydrogen atom, a halogen atom, a nitro group, an alkyl group, an alkoxy group or a phenyl group.

The alkyl group and alkoxy group represented by R⁴ and R⁵ may be of the form of a straight chain or a branched chain without any particular limitation and may, further, have a substituent but, desirably, have 1 to 10 carbon atoms (excluding the carbon atoms of the substituent). As the substituent, further, there can be exemplified those mentioned above for the alkyl groups represented by R¹ to R³. Concrete examples of the alkyl group include methyl group, ethyl group, n- or i-propyl group, n-, i- or t-butyl group, chloromethyl group, trifluoromethyl group, methoxymethyl group, and 1, 1, 1, 3, 3,3-hexafluoro-2-methoxy-2-propyl group, and concrete examples of the alkoxy group include methoxy group, ethoxy group, 1- or 2-propoxy group, 1- or 2-butoxy group, 1-, 2- or 3-octyloxy group and chloromethoxy group.

Further, phenyl groups represented by R⁴ and R⁵ may have a substituent which may be those exemplified above for the aryl groups represented by R¹ to R³.

In the above general formula (1), further, L⁺ is a metal cation, a tertiary or quaternary ammonium ion, a quaternary pyridinium ion, a quaternary quinolinium ion or a quaternary phosphonium ion.

As the metal cations, there can be preferably used alkali metal cations such as sodium ions, lithium ions or potassium ions, or alkaline earth metal cations such as magnesium ions. As the tertiary or quaternary ammonium ions, there can be exemplified tetrabutylammonium ions, tetramethylammonium ions, tetraethylammonium ions, tributylammonium ions and triethanolammonium ions. As the quaternary quinolinium ions, there can be exemplified methylquinolinium ions, ethylquinolinium ions and butylquinolinium ions. As the quaternary phosphonium ions, further, there can be exemplified tetrabutylphosphonium ions and methyltriphenylphosphonium ions.

The aryl borate compound represented by the above formula (2) preferably has an aryl group in a molecule thereof, has two aryl groups in a molecule thereof, has three aryl groups in a molecule thereof or has four aryl groups in a molecule thereof. The following compounds are concrete examples thereof.

The following boron compound salts are concrete examples of the aryl borate compound having an aryl group in a molecule thereof. Examples of boron compounds:
trialkylphenylboron,
trialkyl(p-chlorophenyl)boron,
trialkyl(p-fluorophenyl)boron,
trialkyl(3,5-bistrifluoromethyl)phenylboron,
trialkyl[3,5-bis(1,1,1,3,3,3-hexafluoro-2-methoxy-2-propyl)phenyl]boron,
trialkyl(p-nitrophenyl)boron,
trialkyl(m-nitrophenyl)boron,
trialkyl(p-butylphenyl)boron,
trialkyl(m-butylphenyl)boron,
trialkyl(p-butyloxyphenyl)boron,
trialkyl(m-butyloxyphenyl)boron,
trialkyl(p-octyloxyphenyl)boron,
trialkyl(m-octyloxyphenyl)boron, etc.
(the above alkyl group is n-butyl group, n-octyl group,
n-dodecyl group, or the like group.)

As the boron compound salt, further, there can be exemplified sodium salt, lithium salt, potassium salt, magnesium salt, tetrabutylammonium salt, tetramethylammonium salt, tetraethylammonium salt, tributylamine salt, triethanolamine salt, methylpyridinium salt, ethylpyridinium salt, butylpyridinium salt, methylquinolinium salt, ethylquinolinium salt and butylquinolinium salt.

The following boron compound salts are concrete examples of the aryl borate compound having two aryl groups in a molecule thereof. Examples of boron compounds:
dialkyldiphenylboron,
dialkyldi(p-chlorophenyl)boron,
dialkyldi(p-fluorophenyl)boron,
dialkyldi(3,5-bistrifluoromethyl)phenylboron,
dialkyldi[3,5-bis(1,1,1,3,3,3-hexafluoro-2-methoxy-2-propyl)phenyl]boron,
dialkyl(p-nitrophenyl)boron,
dialkyldi(m-nitrophenyl)boron,
dialkyldi(p-butylphenyl)boron,
dialkyldi(m-butylphenyl)boron,
dialkyldi(p-butyloxyphenyl)boron,
dialkyldi(m-butyloxyphenyl)boron,
dialkyldi(p-octyloxyphenyl)boron,
dialkyldi(m-octyloxyphenyl)boron, etc.
(the above alkyl group is n-butyl group, n-octyl group,
n-dodecyl group, or the like group.)

As the salt thereof, further, there can be exemplified those mentioned above for the aryl borate compound having an aryl group in a molecule thereof.

The following boron compound salts are concrete examples of the aryl borate compound having three aryl groups in a molecule thereof. Examples of boron compounds:
monoalkyltriphenylboron,
monoalkyltris(p-chlorophenyl)boron,
monoalkyltris(p-fluorophenyl)boron,
monoalkyltris(3,5-bistrifluoromethyl)phenylboron,
monoalkyltris[3,5-bis(1,1,1,3,3,3-hexafluoro-2-methoxy-2-propyl)phenyl]boron,
monoalkyltris(p-nitrophenyl)boron,
monoalkyltris(m-nitrophenyl)boron,
monoalkyltris(p-butylphenyl)boron,
monoalkyltris(m-butylphenyl)boron,
monoalkyltris(p-butyloxyphenyl)boron,
monoalkyltris(m-butyloxyphenyl)boron,
monoalkyltris(p-octyloxyphenyl)boron,
monoalkyltris(m-octyloxyphenyl)boron, etc.
(the above alkyl group is n-butyl group, n-octyl group,
n-dodecyl group, or the like group.)

As the salt thereof, further, there can be exemplified those mentioned above for the aryl borate compound having an aryl group in a molecule thereof.

The following boron compound salts are concrete examples of the aryl borate compound having four aryl groups in a molecule thereof.
Examples of boron compounds:
tetraphenylboron,
tetrakis(p-chlorophenyl)boron,
tetrakis(p-fluorophenyl)boron,
tetrakis(3,5-bistrifluoromethyl)phenylboron,
tetrakis[3,5-bis(1,1,1,3,3,3-hexafluoro-2-methoxy-2-propyl)phenyl]boron,
tetrakis(p-nitrophenyl)boron,
tetrakis(m-nitrophenyl)boron,
tetrakis(p-butylphenyl)boron,
tetrakis(m-butylphenyl)boron,
tetrakis(p-butyloxyphenyl)boron,
tetrakis(m-butyloxyphenyl)boron,
tetrakis(p-octyloxyphenyl)boron,
tetrakis(m-octyloxyphenyl)boron, etc.
(the above alkyl group is n-butyl group, n-octyl group, n-dodecyl group, or the like group.)

As the salt thereof, further, there can be exemplified those mentioned above for the aryl borate compound having an aryl group in a molecule thereof.

In the present invention, it is desired to use an aryl borate compound having three or four aryl groups in a molecule thereof from the standpoint of storage stability, and it is most desired to use an aryl borate compound having four aryl groups from the standpoint of easy handling. The above aryl borate compounds can be used in one kind or in two or more kinds being mixed together.

### {B} Acid compounds:

The acid compound is used as a proton source and reacts with the aryl borate compound to form an arylborane.

As the acid compound, there is used an acid group-containing polymerizable momomer which by itself is polymerizable.

Representative examples of inorganic acids in general include hydrochloric acid, sulfuric acid, nitric acid and phosphoric acid, while representative examples of the organic acid include carboxylic acids such as acetic acid, propionic acid, maleic acid, fumaric acid, phthalic acid, benzoic acid, trichloroacetic acid, trifluoroacetic acid, citric acid and trimellitic acid; sulfonic acids such as p-toluenesulfonic acid, benzenesulfonic acid, methanesulfonic acid and trifluoromethanesulfonic acid; and phosphoric acids such as methylphosphonic acid, phenylphosphonic acid, dimethylphosphinic aid and diphenylphosphinic acid.

The acid group-containing polymerizable monomer is a compound having at least one acid group and at least one polymerizable unsaturated group in a molecule thereof, undergoes the polymerization by itself, and does not leak out by the polymerization and curing. In the invention, therefore, the acid group-containing polymerizable monomer is most desirably used as the acid compound. As the acid group which the acid group-containing polymerizable monomer has in its molecule, there can be exemplified the following groups.

### Examples of acid group:

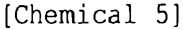

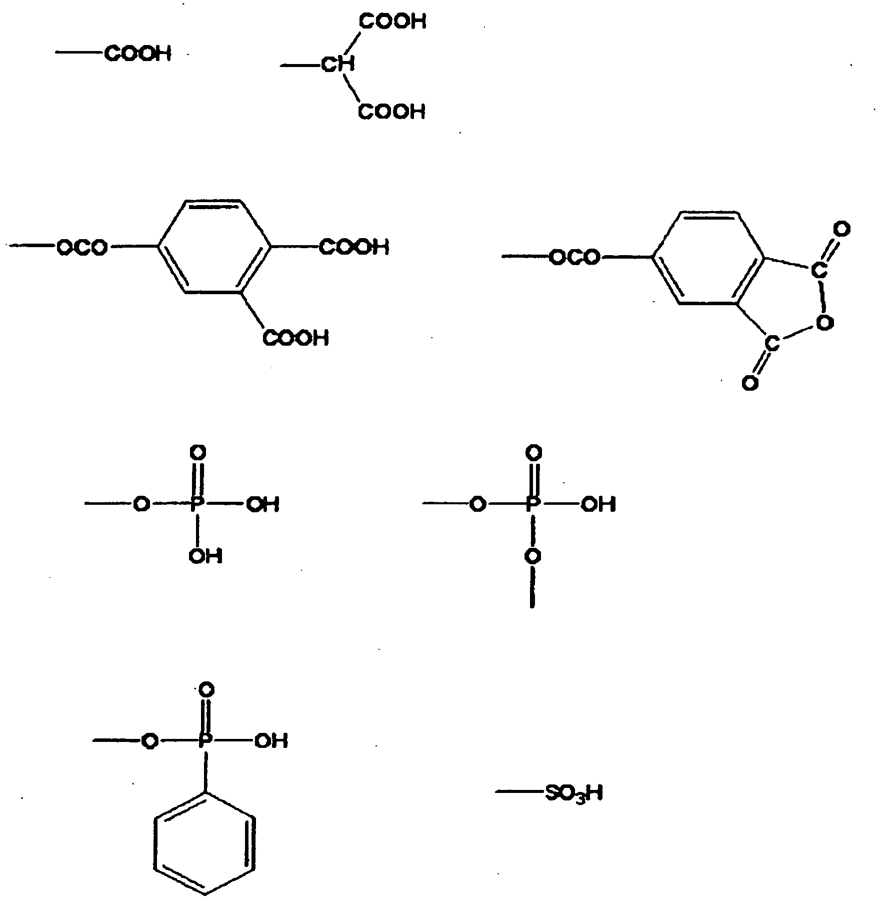

As the polymerizable unsaturated group which the acid group-containing polymerizable monomer has in its molecule, further, there can be exemplified acryloyl group, methacryloyl group, acrylamide group, methacrylamide group, vinyl group, allyl group, ethenyl group and styryl group.

The compounds represented by the following formulas are concrete and representative examples of the acid group-containing polymerizable monomer used in the invention.

Representative examples of the acid group-containing polymer: 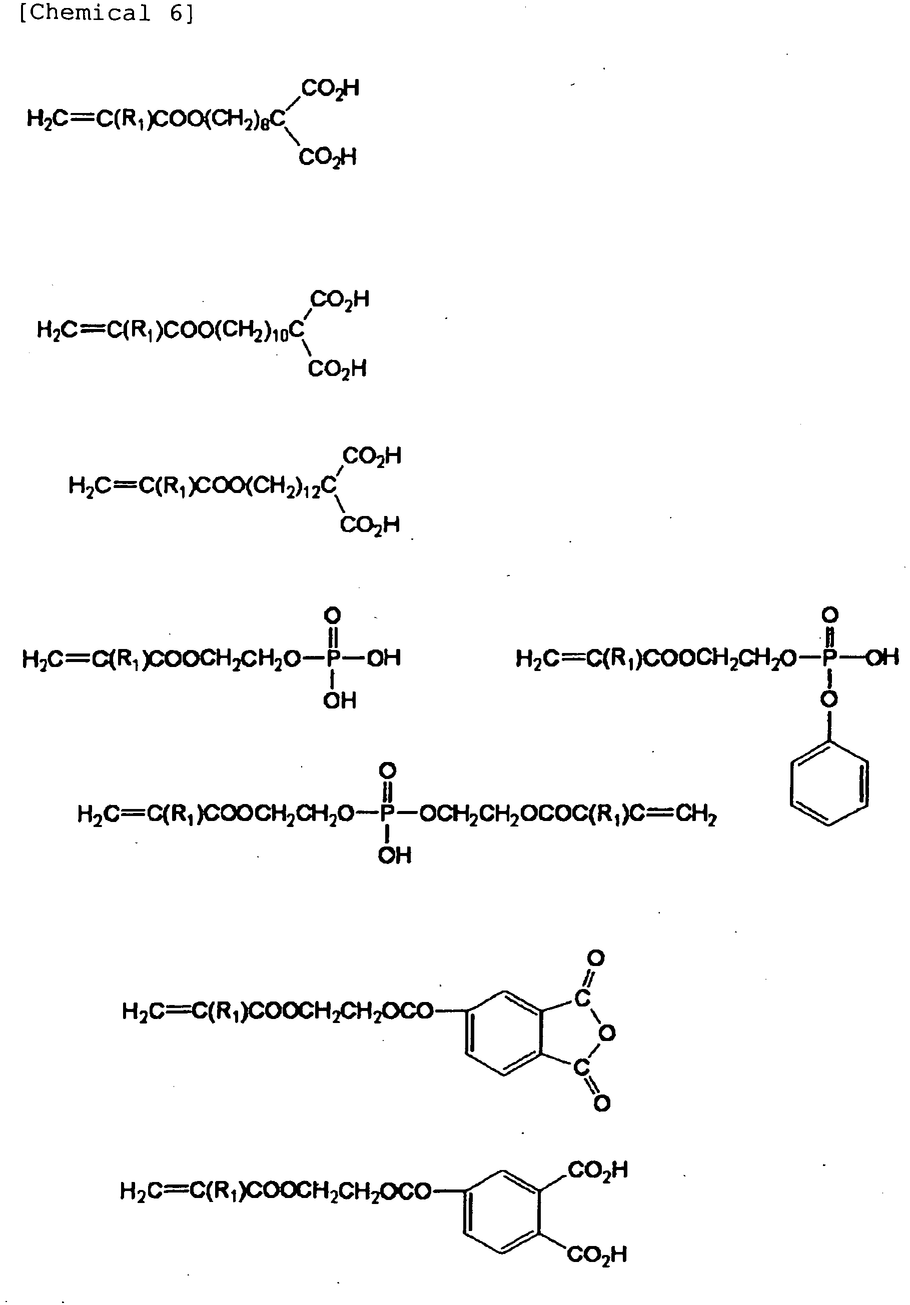 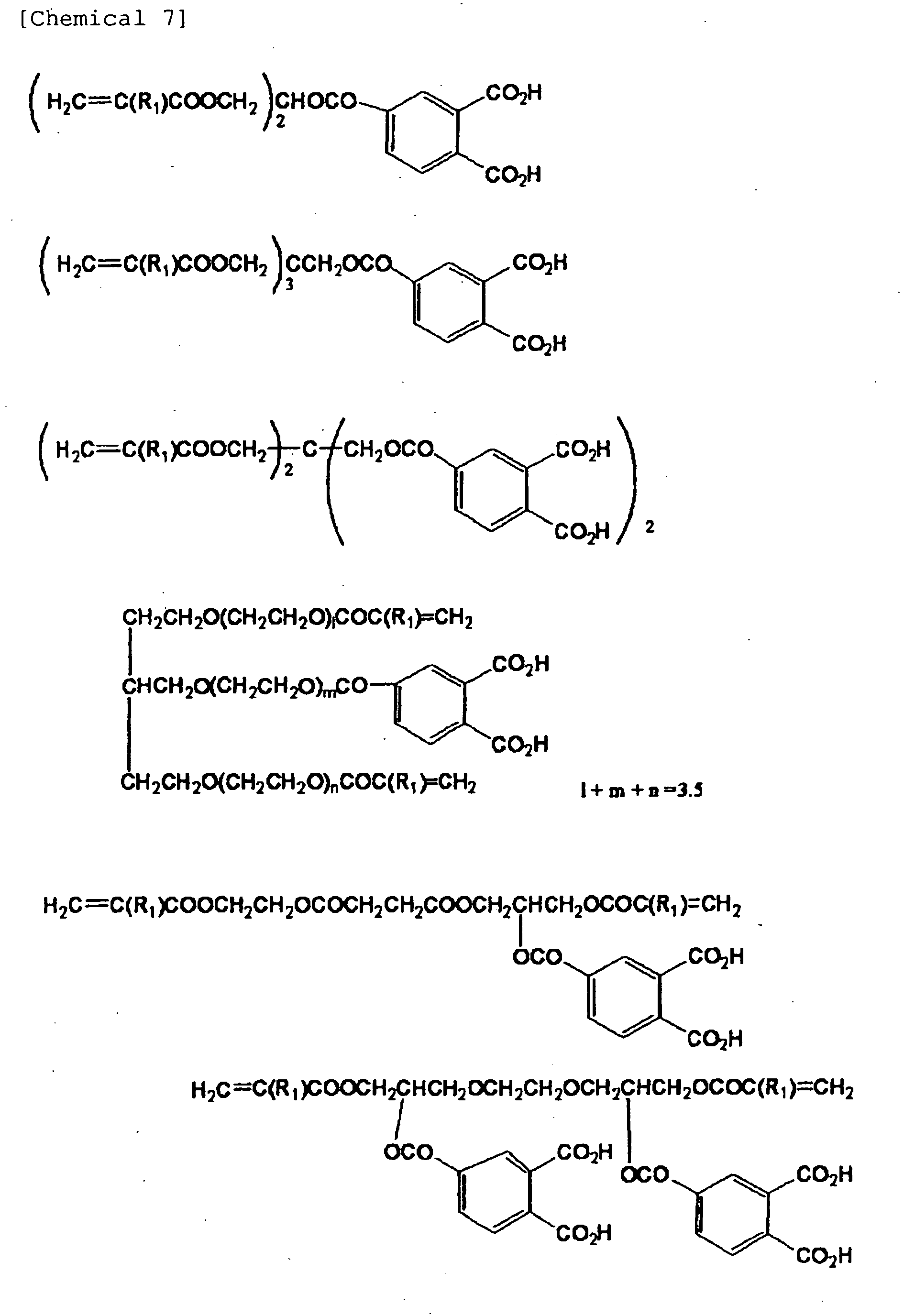 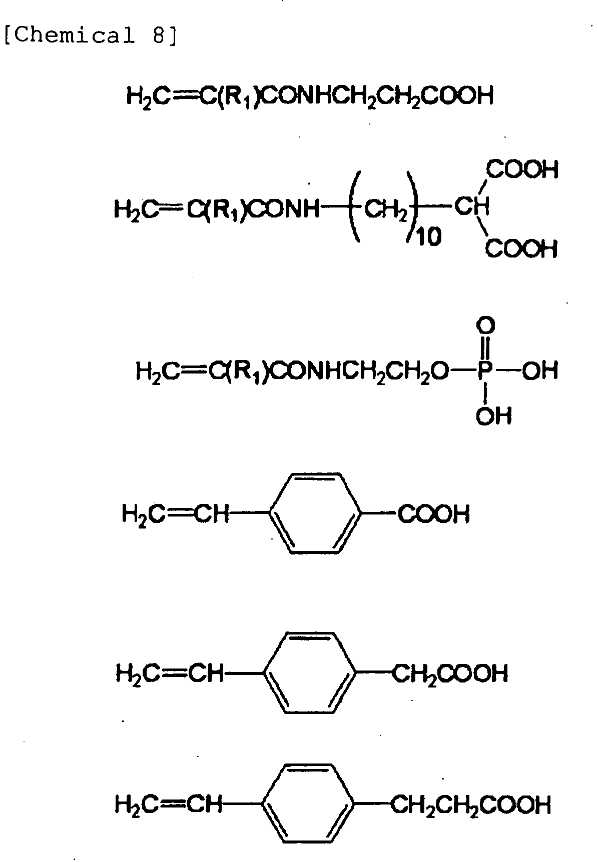 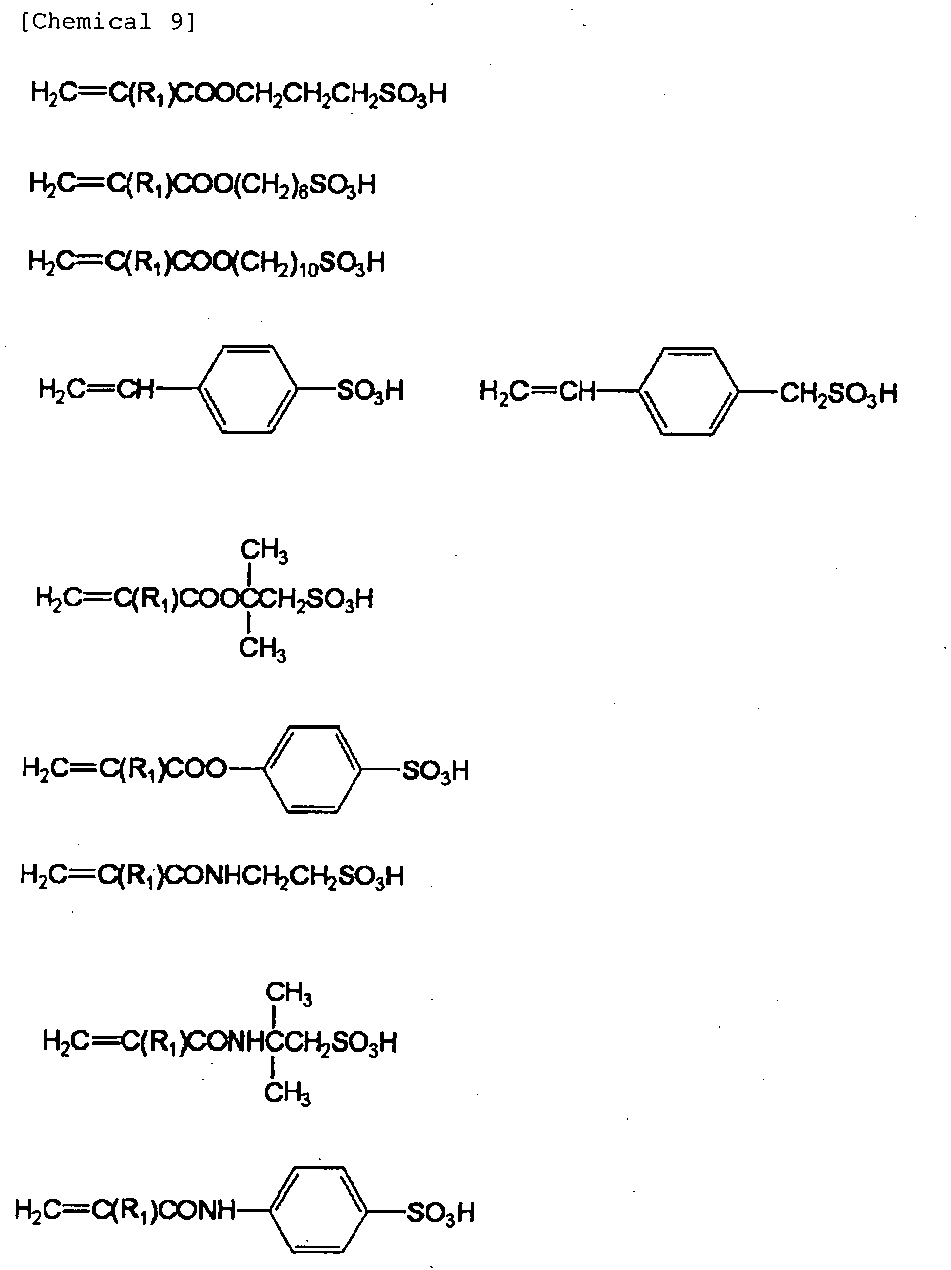 wherein R¹ is a hydrogen atom or a methyl group.

In addition to the above compounds, it is allowable to use vinylphosphonic acids in which a phosphonic acid group is directly bonded to a vinyl group, or to use acrylic acid, methacrylic acid or vinylsulfonic acid as the acid group-containing polymerizable monomer, i.e., as the acid compound which is the component (B).

Among the above acid group-containing polymerizable monomers, it is desired to use a compound having acryloyl group, methacryloyl group, acrylamide group or methacrylamide group as the polymerizable unsaturated group from such a standpoint that the compound is highly capable of being polymerized and cured by itself.

### (C) Vanadium compounds having a valency of +IV or +V:

The vanadium compound having a valency of +IV or +V used in the invention is a compound that works as a decomposition accelerator for accelerating the decomposition of the arylborane such as triphenylborane which is formed by the reaction of an aryl borate compound with an acid compound so that benzo radicals are quickly formed. This compound effectively accelerates the decomposition of arylborane particularly when an organic peroxide (component E) that will be described later is present together with the arylborane.

The vanadium compound assumes an oxidation number or valency of from -I to +V, and the vanadium compound used in the invention has a valency of +IV or +V. The compounds having valencies of -1 to +1 have poor stability whereas the compounds having valencies of +II and +III have low activity exhibiting unsatisfactory decomposition accelerating function and making it difficult to improve the polymerizing activity to a sufficient degree. In the present invention, there is no particular limitation on the vanadium compound having a valency of +IV or +V used as the component (C). Usually, there are used divanadium tetroxide (IV), vanadium oxide acetylacetonato (IV), vanadyl oxalate (IV), vanadyl sulfate (IV), oxobis(1-phenyl-1,3-butanedionate)vanadium (IV), bis(maltolato)oxovanadium (IV), vanadium pentoxide (V), sodium metavanadate (V) and ammon metavanadate (V) in one kind or in two or more kinds in combination.

### (D) Phenol type compounds:

In the invention as described already, the phenol type compound used as the component (D) is a component that serves as a skeleton of the invention for improving the polymerizing activity without impairing the storage stability of the chemical polymerization catalyst, i.e., is a compound represented by the following general formula (1), 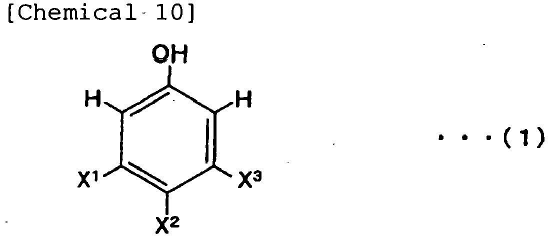 wherein X¹, X² and X³ are each a hydrogen atom or a substitutent selected from the group consisting of a hydroxyl group, an alkoxy group and an alkyl group, and wherein the phenol type compound is not the compound in which X¹ and X³ are hydrogen atoms and X² is a methoxy group.

As will be understood from the above general formula (1) , the phenol type compound is different from the hindered phenols such as BHTs that are usually used as polymerization inhibitors with respect to that there are no substituents at positions (second and sixth positions) neighboring the phenolic hydroxyl group and that the steric hindrance is low to the phenolic hydroxyl group. Namely, in the invention, the steric hindrance to the phenolic hydroxyl group is lean and, therefore, a high activity is obtained exhibiting improved polymerizing activity that is not quite observed when a hindered phenol such as BHT is used. As described above, it is presumed that the polymerizing activity is improved due to improved activity of the vanadium compound which is the component (C) stemming from the exchange of ligands, accelerating the decomposition of the arylborane such as triphenylborane into benzo radicals.

. In the general formula (1) representing the phenol type compound, X¹, X² and X³ are each a hydrogen atom or a substitutent, the substituent being a hydroxyl group, an alkoxy group or an alkyl group. Among these substituents, the alkoxy group and the alkyl group, desirably, have small numbers of carbon atoms and, for example, not more than 4 carbon atoms and are, further, of the form of a straight chain from the standpoint of lowering the steric hindrance. The alkoxy group and the alkyl group may, further, have a substituent as exemplified above for the alkyl group in the general formula (2) of the above aryl borate compound. Still, from the standpoint of steric hindrance, it is desired that the substituent has a small number of carbon atoms. In particular, it is desired that the alkoxy group and the alkyl group inclusive of the substituent have not more than 4 carbon atoms. In the invention, preferred examples of the alkoxy group and the alkyl group include methoxy group, ethoxy group, n-propoxy group, n-butoxy group, and methyl group, ethyl group, n-propyl group, n-butyl group. Particularly, methoxy group and methyl group are preferred.

In the phenol type compound particularly preferred in the invention, the groups X¹, X² and X³ are, preferably, hydrogen atoms, hydroxyl groups or alkoxy groups and, particularly, phenols (X¹ = X² = X³), or hydroquinones (X¹=X³=H, X²=OH) and, more preferably, phenols and hydroquinones and, most preferably, hydroquinones from the standpoint of having two phenolic hydroxyl groups without steric hindrance.

### (E) organic peroxides:

Preferably, an organic peroxide may be blended in addition to the above-mentioned components (A) to (D). Like the above-mentioned vanadium compound which is the component (C), the organic peroxide, too, accelerates the decomposition of the arylborane formed from the aryl borate. Use of the organic peroxide further enhances the polymerizing activity of the catalyst.

As representative organic peroxides that can be used in the invention, there can be exemplified a variety of organic peroxides that are classified into ketone peroxide, peroxyketal, hydroperoxide, diaryl peroxide, peroxy ester, diacyl peroxide and peroxy dicarbonate. Described below are concrete examples of these organic peroxides.

Ketone peroxides:
methyl ethyl ketone peroxide,
cyclohexanone peroxide,
methylcyclohexanone peroxide,
methylacetoacetate peroxide,
acetylacetone peroxide, etc.

Peroxyketals:
1, 1-bis(t-hexylperoxy)3,3,5-trimethylcyclohexane,
1,1-bis(t-hexylperoxy)cyclohexane,
1,1-bis(t-butylperoxy)3,3,5-trimethylcyclohexane,
1,1-bis(t-butylperoxy)cyclohexane,
1,1-bis(t-butylperoxy)cyclododecane,
2,2-bis(t-butylperoxy)butane,
n-buty 4,4-bis(t-butylperoxy)valerate,
2,2-bis(4,4-di-t-butylperoxycyclohexyl)propane, etc.

Hydroperoxides:
P-menthane hydroperoxide,
diisopropylbenzene hydroperoxide,
1,1,3,3-tetramethylbutyl hydroperoxide, cumene hydroperoxide,
t-hexyl hydroperoxide,
t-butyl hydroperoxide, etc.

Dialky peroxides:
*α, α*-bis(t-butylperoxy)diisopropybenzene, dicumyl peroxide,
2,5-dimethyl-2,5-bis(t-butylperoxy)hexane, t-butylcumyl peroxide,
di-t-butyl peroxide,
2,5-dimethyl-2,5-bis(t-butylperoxy)hexyne-3, etc.

Diacyl peroxides:
isobutyl peroxide,
2,4-dichlorobenzoyl peroxide,
3,5,5-trimethylhexanoyl peroxide,
octanoyl peroxide,
lauroyl peroxide,
stearyl peroxide,
succinic acid peroxide,
m-toluoylbenzoyl peroxide,
benzoyl peroxide, etc.

Peroxy dicarbonates:
di-n-propylperoxy dicarbonate,
diisopropylperoy dicarbonate,
bis(4-t-butylcyclohexyl)peroxy dicarbonate,
di-2-ethoxyethylperoxy dicarbonate,
di-2-ethoxyhexylperoxy dicarbonate,
di-2-methoxybutylperoxy dicarbonate,
di(3-methyl-3-methoxybutyl)peroxy dicarbonate, etc.

Peroxy esters:
*α*, *α*-bis(neodecanoylperoxy)diisopropylbenzene, cumylperoxy neodecanoate,
1,1,3,3-tetramethylbutylperoxy neodecanoate,
1-cyclohexyl-1-methylethylperoxy neodecanoate,
t-hexylperoxy neodecanoate,
t-butylperoxy neodecanoate,
t-hexylperoxy pivalate,
t-butylperoxy pivalate,
1,1,3,3-tetramethylbutylperoxy-2-ethyl hexanoate,
2,5-dimethyl-2,5-bis(2-ethylhexanoylperoxy)hexane,
1-cyclohexyl-1-methylethylperoxy-2-ethy hexanoate,
t-hexylperoxy 2-ethyl hexanoate,
t-butylperoxy 2-ethy hexanoate,
t-butylperoxy isobutylate,
t-hexylperoxyisopropyl monocarbonate,
t-butylperoxymaleic acid,
t-butylperoxy 3,5,5-trimethyl hexanoate,
t-butylperoxy laurate,
2,5-dimethyl-2,5-bis(m-toluoylperoxy)hexane,
t-butylperoxyisopropyl monocarbonate,
t-butylperoxy 2-ethylhexyl monocarbonate,
t-hexylperoxy benzoate,
2,5-dimethyl-2,5-bis(benzoylperoxy)hexane,
t-butylperoxy acetate,
t-butylperoxy-m-toluoyl benzoate,
t-butylperoxy benzoate,
bis(t-butylperoxy)isophthalate, etc.

In addition to the above organic peroxides, there can be preferably used t-butyltrimethylsilyl peroxide, 3,3',4,4'-tetra(t-butylperoxycarbonyl)benzophenone and the like.

Among the above organic peroxides, the present disclosure desirably uses ketone peroxides, peroxy esters or diacyl peroxides from the standpoint of polymerizing activity. Among them, it is further desired to use organic peroxides having a 10-hour half-value period temperature of not lower than 60°C from the standpoint of storage stability of the curable composition.

### Composition of the chemical polymerization catalyst:

In the invention, the above components (A) to (D) and the suitably used component (E) can be used each in one kind or in a combination of two or more kinds. The amounts of the components that are used may differ depending upon their kinds. From the standpoint of securing excellent polymerizing activity and favorable storage stability, however, the acid compound (B) is used in an amount of 0.1 to 100 mols and, particularly, 0.5 to 50 mols per mole of the aryl borate compound (A) and the vanadium compound (C) is used in an amount of 0.0005 to 0.015 mol and, particularly, 0.001 to 0.01 mol per mole of the aryl borate compound (A) . The phenol type compound (D) is used in an amount of 0.05 to 200 mols and, particularly, 0.1 to 100 mols per mol of the vanadium compound (C) . It is, further, desired that the organic peroxide (E) is used in an amount of 0.1 to 10 mols and, particularly, 0.5 to 5 mols per mol of the aryl borate compound. Upon using the phenol compound (D) , in particular, the chemical polymerization catalyst of the invention exhibits very high polymerizing activity despite of using the vanadium compound (C) in small amounts. Further, upon using the phenol type compound and the vanadium compound (C) in a small amount, the chemical polymerization catalyst has very excellent storage stability and, further, excels in regard to imparting no color to the cured body.

### Form of storing the chemical polymerization catalyst:

The chemical polymerization catalyst of the invention is stored in a manner that the above-mentioned components are divided into groups which are packaged in a form being mixed with a suitable amount of polymerizable monomer. At the time of use, the components as well as the polymerizable monomer are mixed together so as to exhibit the function of the polymerization catalyst in a predetermined curable composition so as to be used for the dental therapy. Concretely, the aryl borate compound (A) and the acid compound (B) are stored in separate packages. The vanadium compound (C) and the phenol type compound (D) are mixed with the acid compound and are stored together with the polymerizable monomer in a sealed container, while the aryl borate compound (A) is stored together with the polymerizable monomer in a separate sealed container. The organic peroxide (E) is, usually, stored in the same sealed container as that of the aryl borate compound (A).

### <Curable composition>

To use the above chemical polymerization catalyst according to the present invention, the components are taken out from the containers and are mixed together and are put to the use as a curable composition usually in the form of a liquid or a paste. The curable composition undergoes the polymerization and curing without being irradiated with light and forms a cured body without being colored and, further, offers a suitable degree of operation time for executing the filling and molding operations and is, therefore, easy to handle. Further, the chemical polymerization catalyst of the invention is completely polymerized and cured in a period of time shorter than that of the cases of using conventional chemical polymerization catalysts, and is very suited for use in the field of dental therapy where the teeth are restored by filling the curable composition in the damaged portions of the teeth.

That is, the above curable composition contains the polymerizable monomer (the polymerizable monomer does not contain the acid group, and does not serve as a catalyst component of the chemical polymerization catalyst) in addition to the above chemical polymerization catalyst and, further, contains such components as a photo or thermal polymerization initiator and a filler depending upon the use of the curable composition. In this case, the polymerizable monomer is stored in both of the two packages that are separately storing the chemical catalyst components, the polymerizable monomer being mixed therein at suitable ratios. Other components that are used as required are stored, depending on their kinds, in either package that stores the chemical polymerization catalyst component (A) or (B) , or are stored in both packages. Depending upon the cases, however, the other components are stored in a separate package and, at the time of use, are mixed with the components stored in other packages.

### 1. Polymerizable monomers:

The polymerizable monomer is mixed with the components of the chemical polymerization catalyst to prepare the curable composition. Usually, the polymerizable monomer is used in such an amount that the content of the aryl borate compound (A) in the chemical polymerization catalyst is 0.01 to 10 parts by mass and, particularly, 0.1 to 5 parts by mass per 100 parts by mass of the total amount of the polymerizable monomer (without including acid group-containing polymer). As the polymerizable monomer, there can be used any known polymerizable monomer that can be used in combination with a dental chemical polymerization catalyst that has heretofore been used in the dental field without limitation. From the standpoint of curing rate, however, it is desired to use a (meth)acrylate type monomer.

As the (meth) acrylate type polymerizable monomer, there can be exemplified the following mono(meth)acrylate monomers and polyfunctional (meth)acrylate monomers, which may be used in one kind or in a combination of two or more kinds.

Mono(meth)acrylate monomers:
methyl (meth)acrylate,
ethyl (meth)acrylate,
butyl (meth)acrylate
glycidyl (meth)acrylate,
2-cyanomethyl (meth)acrylate,
benzyl (meth)acrylate,
polyethylene glycol mono(meth)acrylate,
allyl (meth)acrylate,
2-hydroxyethyl (meth)acrylate,
3-hydroxypropyl (meth)acrylate,
glyceryl mono(meth)acrylate, etc.

Polyfunctional (meth)acrylate type monomers:
ethylene glycol di(meth)acrylate,
diethylene glycol di(meth)acrylate,
triethylene glycol di(meth)acrylate,
nonaethylene glycol di(meth)acrylate,
propylene glycol di(meth)acrylate,
dipropylene glycol di(meth)acrylate,
2,2'-bis[4-(meth)acryloyloxyethoxyphenyl]propane,
2,2'-bis[4-(meth)acryloyloxyethoxyethoxyphenyl] propane,
2,2'-bis[4-(meth)acryloyloxyethoxyethoxyethoxyethoxy ethoxyphenyl]propane,
2,2'-bis{4-[3-(meth)acryloyloxy-2-hydroxypropoxy]-phenyl}propane,
1,4-butanediol di(meth)acrylate,
1,6-hexanediol di(meth)acrylate,
1,9-nonanediol di(meth)acrylate,
trimethylolpropane tri(meth)acrylate,
neopentyl glycol di(meth)acrylate,
urethane (meth)acrylate,
epoxy (meth)acrylate, etc.

In the dental curable composition, it is further allowable to execute the polymerization by mixing a polymerizable monomer other than the above (meth)acrylate type monomer in order to adjust the viscosity of the curable composition or to adjust properties depending upon the use of the curable composition. Here, however, it is desired that the ratio of blending the (meth) acrylate type monomer is not less than 50% by weight and, desirably, not less than 60% by weight in the whole polymerizable monomers (excluding the acid group-containing polymer used as the catalyst component). If the amount thereof is less than 50% by weight, it becomes difficult to attain a rate of curing required in the dental clinic.

As the other polymerizable monomer to be used in combination with the above (meth)acrylate type monomer, there is used, for example, a sulfur atom-containing polymerizable monomer. The sulfur atom-containing polymerizable monomer has its sulfur atom to bond to a metal atom and has been known to be an adhesive component effective for a dental prosthetic made of a noble metal used for restoring a damaged tooth. That is, when the curable composition is used as an adhesive or primer for a dental prosthetic, it is desired to use the sulfur atom-containing polymerizable monomer in combination with the (meth)acrylate type monomer.

As the above sulfur atom-containing polymerizable monomer, there have been known disclosed in, for example, JP-A-2000-248201 or, concretely, radically polymerizable compounds capable of forming a mercapto group (SH) by tautomerism as expressed by the following general formulas (3a) to (3e), radically polymerizable disulfide compound as expressed by the following general formulas (3f) to (3i), and radically polymerizable thioether compounds as expressed by the following general formulas (3j) to (3k).

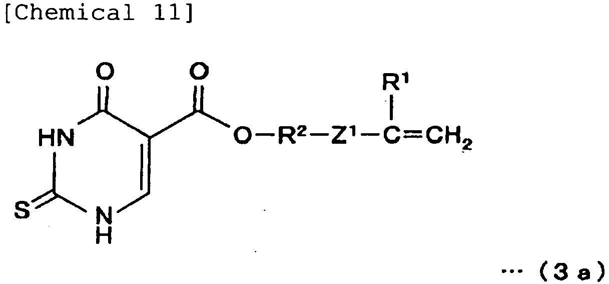

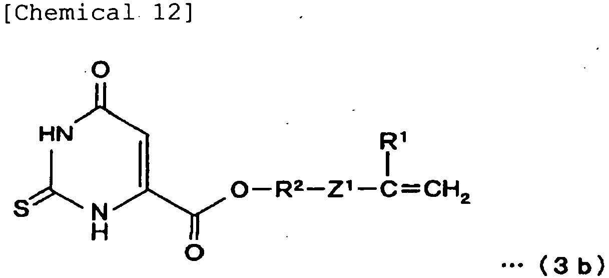

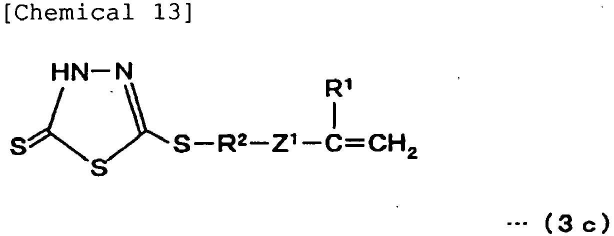

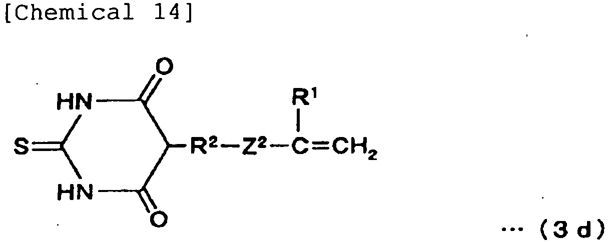

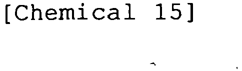

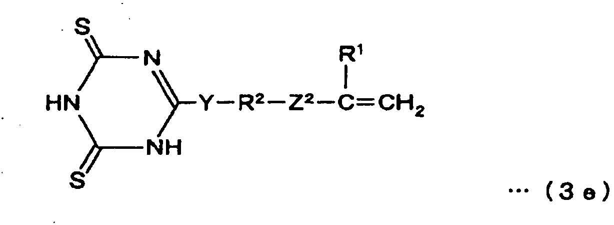

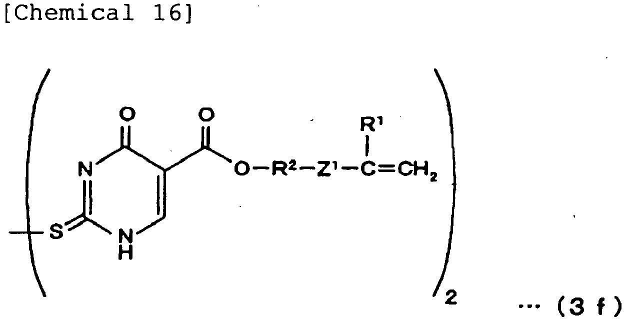

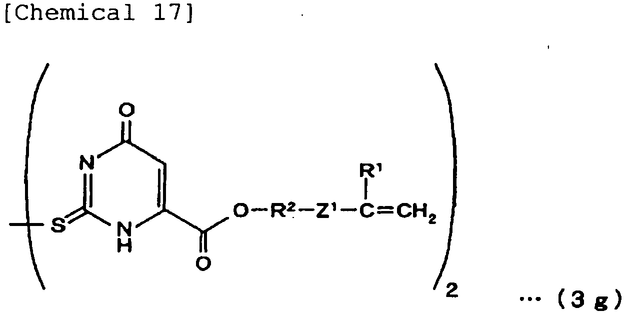

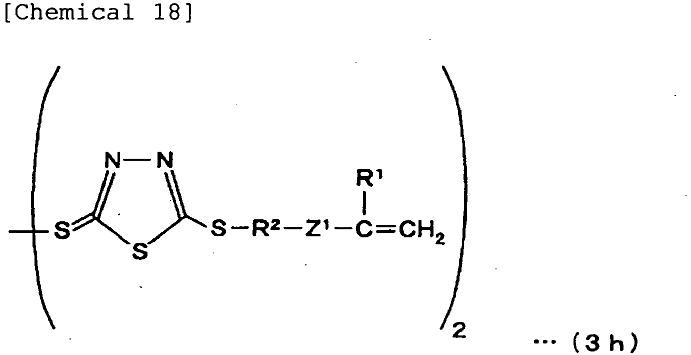

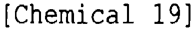

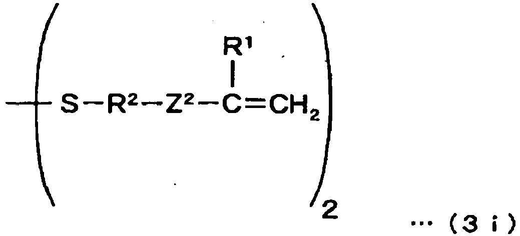

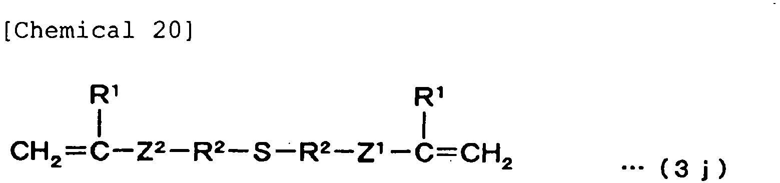

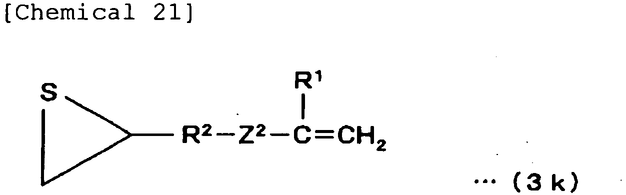

In the above formulas (3a) to (3k),
R¹ is a hydrogen atom or a methyl group,
R² is an alkylene group having 1 to 12 carbon atoms, a group -CH₂-C₆H₄-CH₂ or a group - (CH₂) p-Si (CH₃)₂-(CH₂)q- (wherein p and q are integers of 1 to 5),
Z¹. is a group -O-CO-, a group -OCH₂- or a group -OCH₂-C₆H₄-,
Z² is a group -O-CO-, a group -C₆H₄- or a bonding hand (in which the group R² and an unsaturated carbon atom are directly bonded together), and
Y is -S-, -O- or -N(R')- (wherein R' is a hydrogen atom or an alkyl group having 1 to 5 carbon atoms).

As the other polymerizable monomer used for adjusting properties such as viscosity, etc., there can be representatively used styrene or derivatives thereof, fumaric acid ester, allyl compound, epoxy compound, oxetane compound, and vinyl ether compound. Concretely, the following compounds can be exemplified.

Styrenes or derivatives thereof:
styrene,
p-chlorostyrene,
p-hydroxystyrene,
divinylbenzene,
*α*-methylstyrene, etc.

Fumaric acid esters:
monomethyl fumarate,
diethyl fumarate,
diphenyl fumarate, etc.

Allyl compounds:
diallyl phthalate,
diallyl terephthalate,
diallyl carbonate,
allyl diglycol carbonate, etc.

Epoxy compounds:
diglycerol polyglycidyl ether,
pentaerythritol polyglycidyl ether,
1,4-bis(2,3-epoxypropoxyperfluoroisopropyl) cyclohexane,
sorbitol polyglycidyl ether,
trimethylolpropane polyglycidyl ether,
resorcin diglycidyl ether,
1,6-hexanediol diglycidyl ether,
polyethylene glycol diglycidyl ether,
phenyl glycidyl ether,
p-tert-butylphenyl glycidyl ether,
adipic acid diglycidyl ether,
o-phthalic acid diglycidyl ether,
dibromophenyl glycidyl ether,
1,2,7,8-diepoxyoctane, ,
4,4'-bis(2,3-epoxypropoxyperfluoroisopropyl)diphenyl ether,
3,4-epoxycyclohexylmethyl-3' ,4'-epoxycyclohexane carboxylate,
3,4-epoxycyclohexyloxylane,
ethylene glycol bis(3,4-epoxycyclohexane carboxylate), etc.

Oxetane compounds:
3-ethyl-3-hydroxymethyloxetane,
3-ethyl-3-(phenoxymethyl)oxetane,
3-ethyl-3-(naphthoxymethyl)oxetane,
di[1-ethyl(3-oxetanyl)jmethyl ether,
3-ethyl-3-(2-ethylhexyloxymethyl)oxetane,
1,4-bis{[(3-ethyl-3-oxetanyl)methoxy]methyl}benzene, etc.

Vinyl ether compound:
vinyl-2-chloroethyl ether,
vinyl-n-butyl ether,
triethylene glycol divinyl ether,
1,4-cyclohexanedimethanoldivinyl ether,
trimethylolethanetrivinyl ether,
vinylglycidyl ether, etc. .

Among the other polymerizable monomers exemplified above, the epoxy compound, oxetane compound and vinyl ether compounds are cationically polymerizable monomers that start polymerizing with the acid compound which is the component (B) in the dental chemical polymerization catalyst. The above other polymerizable monomers may be used in one kind or in two or more kinds being mixed together.

### 2. Polymerization initiators:

The above curable composition can be blended with a photopolymerization initiator or a thermal polymerization initiator so far as they do not impair the properties of the above chemical polymerization catalyst. With such polymerization initiators being blended, the above chemical polymerization initiator of the invention assists the polymerization and curing when the polymerization and curing are effected by the irradiation with light or by heating. When, for example, the photopolymerization initiator is used in combination, there is realized a so-called dual cure type curable composition capable of undergoing both chemical curing and photo curing, making it possible to quickly conduct the polymerization and curing even in case the irradiation with light is insufficient.

Though there is no particular limitation on the thermal polymerization initiator, there is preferably used an azo compound such as azobisisobutylonitrile.

The photopolymerization initiator commences the polymerization upon the irradiation with ultraviolet rays or visible light rays, and its representative examples include:
*α*-diketones such as diacetyl, acetylbenzoyl, benzyl, 2,3-pentadione, 2,3-octadione, 4,4'-dimethoxybenzyl, 4,4'-oxybenzyl, camphorquinone, 9,10-phenanthrenequinone and acenaphthenequinone;
benzoinalkyl ethers such as benzoinmethyl ether, benzoinethyl ether and benzoinpropyl ether;
thioxanthone derivatives such as 2,4-diethoxythioxanthone, 2-chlorothioxanthone and methylthioxanthone;
benzophenone derivatives such as benzophenone, p,p'-dimethylaminobenzophenone and p,p'-methoxybenzophenone; and
acylphosphinoxide derivatives such as 2, 4, 6-trimethylbenzoyldiphenyl phosphinoxide, bis(2,6-dimethoxybenzoyl)-2,4,4-trimethylpentyl phosphinoxide and bis(2,4,6-trimethylbenzoyl)phenyl phosphinoxide.

In particular, *α*-diketone and acyl phosphinoxide are preferred.

When the above photopolymerization initiator is used, further, there can be used an amine compound as a promotor. The amine compound used in this case, however, will be the one that does not neutralize the above acid compound (B) to lower its function, such as 4-(N,N-dimethyamino)benzoic acid and its lower alkyl (C1 to C4) esters or 4-dimethylaminoacetophenone.

Use of a dye and a photo acid generator in combination is also desired as a photopolymerization initiator. That is, the chemical polymerization catalyst of the invention contains the aryl borate compound (A) as an essential component and, therefore, a system comprising aryl borate/dye/photo acid generator desirably works as a photopolymerization initiator.

As the dye, there can be exemplified those dyes of the cumarin type. The particularly preferred cumarin type dye has a maximum absorption wavelength in the visible ray region of 400 to 500 nm, and exhibits a high sensitivity to the irradiator that is usually used for the dental applications. Concrete and representative examples of the cumarin type dye include 3-thienoylcumarin, 3,3'-carbonylbis(7-diethylamino)cumarin, 3,3'-carbonylbis(4-cyano-7-diethylamino)cumarin, etc.

The photo acid generator forms Bronsted acid or Lewis acid upon being irradiated with light, and any known photo acid generator can be used without limitation provided it is decomposed by the dye under the irradiation with visible light and generates acid. As the photo acid generator, there can be particularly preferably used a halomethyl group-substituted s-triazine derivative or a diphenyliodonium salt compound since it is capable of highly efficiently generating acid under the irradiation with visible light. Described below are representative examples of the halomethyl group-substituted s-triazine derivative and diphenyliodonium salt compound.

Halomethyl group-substituted s-triazine derivatives:
2,4,6-tris(trichloromethyl)-s-triazine,
2-methyl-4,6-bis(trichloromethyl)-s-triazine,
2-(2,4-dichlorophenyl)-4,6-bis(trichloromethyl)-s-triazine, etc.

Diphenyliodonium salt compounds:
diphenyliodonium,
p-octyloxyphenylphenyliodonium,
tetrafluoroborate,
hexafluorophosphate,
hexafluoroantimonate,
trifluoromethanesulfonate, etc.

The above polymerization initiators are not only used in a single kind but are also used, as required, in a plurality of kinds in combination. There is no particular limitation on the amount of use of these polymerization initiators provided they are used in a range in which they do not impair the effect of the invention. It is, however, desired that the polymerization initiators are used in amounts of 1 to 1000 parts by weight, particularly, 10 to 500 parts by weight per 100 parts by weight of the aryl borate compound which is the dental chemical polymerization catalyst of the invention.

### 3. Fillers.

The curable composition can be blended with various kinds of fillers depending upon the use thereof. For example, when used as a dental adhesive such as an adhesive (bonding agent) for directly restoring the tooth or an adhesive for indirectly restoring the tooth or is used as a dental restorative that serves as a dental prosthetic, such as composite resin, it is desired that the curable composition of the invention is blended with a filler such as inorganic filler, organic filler or organic/inorganic composite filler.

As the inorganic filler, there can be exemplified quartz, silica, silica-alumina, silica-titania, silica-zirconia, silica-magnesia, silica-calcia, silica-barium oxide, silica-strontium oxide, silica-titania-sodium oxide, silica-titania-potassium oxide, titania, zirconia and alumina. These inorganic fillers are added, as required, with their surfaces being treated with a silane coupling agent such as γ-methacryloxypropyltrimethoxysilane, ε-methacryloxyoctyltrimethoxysilane or vinyltrimethoxysilane.

As the inorganic filler, further, there can be used a polyvalent ion-releasing filler. Upon adding such a filler, the chelate reaction of the acid group-containing polymerizable monomer with polyvalent metal ions proceeds along with the polymerization reaction, and the strength of the cured body can be improved.

As the polyvalent metal ion-releasing filler, there can be exemplified hydroxides such as calcium hydroxide and strontium hydroxide, as well as zinc oxide, silicate glass, fluoroaluminosilicate glass, barium glass and strontium glass. Among them, the fluoroaluminosilicate glass most excels from the standpoint of preventing the cured body from being colored and, therefore, can be desirably used. The fluoroaluminosilicate glass is the one that has been widely used as a dental cement. The fluoroaluminosilicate glass that has been generally known has the following composition as expressed by ionic mass percentage.
Silicon: 10 to 33%, particularly, 15 to 25%
Aluminum: 4 to 30%, particularly, 7 to 20%
Alkaline earth metal: 5 to 36%, particularly, 8 to 28%
Alkali metal: 0 to 10%, particularly, 0 to 10%
Phosphorus: 0.2 to 16%, particularly, 0.5 to 8%
Fluorine: 2 to 40%, particularly, 4 to 40%
Oxygen: balance

In addition to the one of the above composition, there can be used a glass in which part or whole of the alkaline earth metal is substituted by magnesium, strontium or barium. In particular, the one substituted by strontium imparts X-ray impermeable property and large strength to the cured body and is, therefore, often favorably used.

Like the above inorganic filler, the above polyvalent metal ion-eluting filler, too, may be used without any problem by being treated with a surface-treating agent as represented by a silane coupling agent.

As the organic filler, further, there can be exemplified such polymers as polymethyl methacylate, polyethyl methacrylate, methyl methacrylate/ethyl methacrylate copolymer, ethyl methacrylate/butyl methacrylate copolymer, methyl methacrylate/trimethylolpropane methacrylate copolymer, polyvinyl chloride, polystyrene, chlorinated polyethylene, nylon, polysulfone, polyethersulfone, and polycarbonate.

As the organic/inorganic composite filler, there can be exemplified a pulverized composite powder of the .above inorganic oxide (inorganic filler) and the polymer (organic filler).

The particles of the above various kinds of fillers may have any shape without limitation, such as pulverized shape as obtained by an ordinary pulverization or spherical shape. There is no particular limitation, either, on the particle diameter of these fillers. Usually, the particle diameter is not larger than 100 µm and, particularly, not larger than 30 µm from the standpoint of dispersion property.

The amount of the filler that is added may vary depending on its kind. When the above polyvalent metal ion-eluting filler is used, its amount is selected to be in a range of 1 to 20 parts by mass and, particularly, 2 to 15 parts by mass per 100 parts by mass of the whole polymerizable monomer from the standpoint of maximizing the effect for improving the adhesion by ionic crosslinking with the eluted ions. Within the above range, it is desired that other fillers, too, are used in combination. It is, further, desired that the total amount of the filler inclusive of the above polyvalent metal ion-eluting filler is in a range of 50 to 900 parts by mass and, particularly, 100 to 800 parts by mass per 100 parts by mass of the whole polymerizable monomer. If the amount of the filler that is blended is smaller than the above range, physical strength of the cured body is not improved by the addition of the filler to a sufficient degree. If the amount thereof is larger than the above range, the curable composition has decreased fluidity deteriorating the operability which may make it difficult to carry out such operations as applying or filling the curable composition onto a predetermined part or forming the curable composition into a predetermined shape.

### 4. Other blending agents.

In order to further impart desired properties depending on the use, the above curable composition containing the chemical polymerization catalyst may be further blended with known blending agents, such as, water and organic solvent in addition to the above-mentioned materials.

When the curable composition is used, for example, as an adhesive for adhering the dental restorative such as composite resin or prosthetic to the tooth or is used as a primer for adhesion, the water or the organic solvent accelerates the penetration of acid compound in the chemical polymerization catalyst into the tooth (demineralization of tooth) and, further, accelerates the elution of ions from the polyvalent metal ion-eluting filler or the ionic crosslinking, contributing to increasing the strength of adhesion of the cured body to the tooth (particularly, to the enamel).

As the water, there is preferably used de-ionized water or distilled water from the standpoint of storage stability, adaptability to the living body and being substantially free of impurities detrimental to the adhesion. Usually, water is added in an amount in a range of 2 to 30 parts by weight and, particularly, 3 to 20 parts by weight per the total amount of 100 parts by mass of the whole polymerizable monomers. In particular, if water is used in amounts larger than the required amount, storage stability is deteriorated and strength of the cured body decreases. Besides, when the curable composition is applied to the predetermined part, water cannot be easily removed by blowing the air, curing property decreases and strength of adhesion decreases.

As the organic solvent, there can be used a water-soluble organic solvent or water-insoluble organic solvent.

As the water-soluble organic solvent, there can be used alcohols or ethers such as methanol, ethanol, propanol, butanol, ethylene glycol, propanediol, butanediol, pentanediol, butenediol, glycerin, trimethylolpropane, hexanetriol, allyl. alcohol, diethylene glycol, diethylene glycol monomethyl ether, diethylene glycol monoethyl ether, triethylene glycol, triethylene glycol monomethyl ether, tetraethylene glycol, propylene glycol, dipropylene glycol, tripropylene glycol, 2-methoxyethanol, 2-ethoxyethanol, 2-(methoxyethoxy)ethanol, 2-isopropoxyethanol, 2-butoxyethanol, 1-methoxy-2-propanol, 1-ethoxy-2-propanol, dipropylene glycol monomethyl ether, tripropylene glycol monomethyl ether and glycerin ether; ketones such as acetone and methyl ethyl ketone; carboxylic acids such as acetic acid, anhydrous acetic acid and propionic acid.

As the water-insoluble organic solvent, there can be used hexane, heptane, octane, toluene dichloromethane, chloroform, carbon tetrachloride, dichloroethane, trichloroethane, methyl ethyl ketone, pentanone, ethyl formate, propyl formate, butyl formate, ethyl acetate, propyl acetate and butyl acetate. Among them, it is desired to use those that have little beneficial/harmful action to the living body, such as ethanol, propanol, ethylene glycol, propanediol, butanediol, pentanediol, glycerin, trimethylolpropane, hexanetriol, diethylene glycol, diethylene glycol monomethyl ether, diethylene glycol monoethyl ether, triethylene glycol, triethylene glycol monomethyl ether, tetraethylene glycol, propylene glycol, dipropylene glycol, tripropylene glycol, dipropylene glycol monomethyl ether, tripropylene glycol monomethyl ether, glycerin ether, acetone and 2-hydroxyethyl methacrylate.

Among the above various kinds of organic solvents, it is most desired to use, particularly, ethanol, propanol, ethylene glycol, propanediol and acetone. These organic solvents are, usually, used in amounts of 20 to 300 parts by mass and, particularly, 50 to 400 parts by mass per 100 parts by mass of the whole polymerizable monomers.

In addition to the above, it is allowable to use known viscosity-imparting agent, polymerization-adjusting agent, dye, antistatic agent, pigment and perfume in a range in which they do not impair the polymerization activity or storage stability.

Depending upon their kinds, the above blending agents are contained together with the polymerizable monomers in either a package that contains the aryl borate compound (A) which is the chemical polymerization catalyst or in a package that contains the acid compound (B), or are contained in both packages being divided into suitable amounts.

The curable composition blended with the chemical polymerization catalyst features high storage stability in a state where it is stored being packaged in a divided manner, exhibits excellent polymerizing activity when it is put to use by mixing the components together after stored for extended periods of time, not only start polymerizing even without being irradiated with light but also exhibits excellent adhesiveness to the tooth yet effectively suppressing the cured body from developing color. Therefore, the curable composition can be favorably used in the field of dental therapy as a dental restorative for restoring the damaged tooth or an adhesive or a primer for adhering and fixing the dental restorative to a predetermined part.

When used, for example, as the adhesive (bonding material) for directly restoring the tooth, the curable composition is applied onto the tooth surface by using a sponge or a small brush. Immediately thereafter, or after left to stand for several seconds to several minutes, the restorative such as composite resin is filled, and the curable resin is polymerized and cured to strongly adhere the restorative to the tooth. When used as an adhesive (dental cement) for indirectly restoring the tooth, the curable composition is applied onto the tooth surface and/or onto the restorative directly or by using a spatula or a small brush, the two are contacted together, and the curable composition is polymerized and cured. In order to attain a further increased strength of adhesion, in this case, the tooth surface may be treated with an acid aqueous solution or a pre-treating material containing an acid group-containing polymerizable monomer and water and, thereafter, the adhesion may be attained in a manner as described above.

### EXAMPLES

The invention will now be concretely described by way of examples.

The compounds and their abbreviations used in Examples and Comparative Examples are described in (1), the method of measuring the curing time is described in (2), the methods of measuring properties of various curable bodies are described in (3), the method of measuring the strength of adhesion of the adhesives for direct dental restoration is described in (4) and the method of measuring the strength of adhesion of the adhesives for indirect dental restoration is described in (5) .

### (1) Abbreviations and structures.

### [Aryl borate compounds (A)]

PhBNa: tetraphenylborate sodium salt
PhBTEOA: tetraphenylborate triethanolamine salt
PhBDMPT: tetraphenylborate dimethyl-p-toludine salt
PhBDMBE: tetraphenylborate ethyl dimethylaminobenzoate
FPhBNa: sodium tetrakis(p-fluorophenyl)borate
PhBTEA: tetraphenylborate triethylamine salt
PhBDMEM: tetraphenylborate dimethylaminoethyl methacrylate
BFPhBNa: sodium butyltri(p-fluorophenyl)borate

### [Acid group-containing polymerizable monomers (B)]

PM: A mixture of 2-methacryloyloxyethyldihydrogen phosphate and bis(2-methacryloyloxyethyl)hydrogen phosphate
MAC-10: 11-Methacryloyloxy-1,1-undecanedicarboxylic acid
4-META: 4-Methacryloyloxyethyltrimellitic anhydride MMPS: 2-Methacrylamide-2-methylpropanesulfonic acid

### [Polymerizable monomers without acid group]

MMA: Methyl methacrylate
TMPT: Trimethylolpropane trimethacrylate
Bis-GMA: 2,2-Bis(4-(2-hydroxy-3-methacryloxypropoxy) phenyl)propane
3G: Triethylene glycol dimethacrylate
D26E: 2,2-bis[(4-methacryloyloxypolyethoxyphenyl) propane]
HEMA: 2-Hydroxyethyl methacrylate
MTU-6: 6-Methacryloyloxyhexyl 2-thiouracyl-5 carboxylate
NPG: Neopentyl glycol dimethacrylate

### [Vanadium compounds (C)]

VOAA: Vanadium oxide (IV) acetyl acetonato
V205: Vanadium oxide (V)
BMOV: Bis(maltolato)oxovanadium (IV)

### [Phenol type compounds (D)]

HQME: Methoxyhydroquinone
HQ: Hydroquinone
Ph: Phenol

### [Other phenol type compounds]

BHT: 2,6-di-t-butylhydroxytoluene .

### [Organic peroxides (E)]

Percumyl H: Cumene hydroperoxide
Perocta H: 1,1,3,3-Tetramethylbutyl hydroperoxide

### [Fillers]

FASG: Fluoroaluminosilicate glass powder
3Si-Zr: Irregular shaped silica-zirconia of which the surfaces are treated with
γ-methacryloyloxypropyltrimethoxysilane, average particle diameter, 3 µ m
0.3Si-Ti: Spherical silica-titania (or aggregated body thereof) of which the surfaces are treated with γ-methacryloyloxypropyltrimethoxysilane, average particle diameter of primary particles, 0.3 µm
F1: Amorphous silica (treated with methyl trichlorosilane), particle diameter, 0.02 µm

.

### (2) Measuring the curing time.

The curing time was measured relying on the exothermic method by using a thermistor thermometer. That is, 5 g of a polymerizable monomer solution (a) containing an acid compound and 5 g of a polymerizable monomer solution (b) containing an aryl borate compound were stirred and mixed together for 20 seconds to prepare a homogeneous solution thereof. Next, the solution was flown into a Teflon (registered trademark) mold measuring 2 cm x 2 cm x 1 cm having a hole 6 mm in diameter perforated in the center thereof, and the thermistor thermometer was inserted in the hole. The time until a maximum temperature was recorded from the start of mixing was regarded as a curing time.

The measurement was taken in a constant-temperature room maintained at 23°C.

### (3) Evaluating the curing property and stickiness on the surface.

Curable compositions were prepared in the same manner as above, flown into the same mold, and were cured at 23°C for 15 minutes in the air. The cured bodies were evaluated for their hardness and stickiness on the surfaces into five steps.
- ⊚:: Possessed a sufficient degree of hardness without any stickiness on the surface. '
- ○:: Possessed a sufficient degree of hardness having, however, stickiness on the surfaces only.
- Δ:: Jellied and unpolymerized monomers remained on the surfaces.
- X:: Partly jellied.
- XX:: Was not quite cured.

### (4) Method of measuring the strength of adhesion of the adhesive for directly restoring the tooth.

Within 24 hours after the slaughter, a bovine lower jaw foretooth was pulled out, and the enamel surface and the dentin surface were ground by using a #800 emery paper while pouring water so as to be in parallel with the labial face. Next, the compressed air was blown onto the surface for about 10 seconds to dry. Thereafter, a double-sided tape having a hole of 3 mm in diameter perforated therein was fixed to the surface to specify the area of adhesion. Next, a wax of a thickness of 1 mm having a hole of 8 mm in diameter perforated therein was stuck thereto so as to be in concentric the double-sided tape to form a mimic cavity. The dental adhesive for direct restoration prepared just before the use was applied into the mimic cavity and was left to stand for 20 seconds.

In the case of using a photo curable composite resin, the photo curable composite resin [ESTELITE Σ, manufactured by Tokuyama Dental Co.] was filled in the mimic cavity into which the dental adhesive has been applied, and was covered with a polypropylene sheet. Next, the composite resin was polymerized and cured by the irradiation with light for 30 seconds by using the Power-Light [manufactured by Tokuyama Dental Co.] to prepare a test piece. In the case of using a chemically curable composite resin, the chemically curable composite resin [PALFIQUE, manufactured by Tokuyama Dental Co.] was, similarly, filled and was cured to prepare a test piece.

The test pieces prepared as described above were dipped in water of 37°C for 24 hours, and were subjected to the tensile test by using a tensile tester (Autograph AG5000 manufactured by Shimazu Seisakusho Co.) under a condition of a crosshead speed of 1 mm/min. Eight adhesion test pieces were measured per a test, and an average value thereof was regarded to be a strength of adhesion.

### (7) Method of measuring the strength of adhesion of the adhesive for indirectly restoring the tooth.

The area of adhesion was specified in the same manner as in the method of measuring the strength of adhesion of the above adhesive for directly restoring the tooth. Next, the tooth surface-treating material was thinly applied, left to stand for 20 seconds and, thereafter, the compressed air was blown for about 5 seconds to dry. The adhesive for indirect restoration of the invention prepared just before the use was applied onto the tooth surface that has been treated as described above, and a stainless steel attachment of a diameter of 8 mm was press-contacted thereto to prepare a test piece. The test piece was maintained in an atmosphere of a temperature of 37°C and a humidity of 100% for one hour, was dipped in water of 37°C for 24 hours, and was subjected to the tensile test by using the tensile tester (Autograph AG5000 manufactured by Shimazu Seisakusho Co.) at a crosshead speed of 1 mm/min. Eight adhesion test pieces were measured per a test, and an average value thereof was regarded to be a strength of adhesion.

First, the chemical polymerization catalysts were evaluated for their polymerization initiation ability and basic properties of the obtained cured bodies.

### <Example 1>

To 100 parts by mass of a solution of D26E/3G (50 wt%/50 wt%), there were added 5 parts by mass of PM that was an acid compound as the component (B), 0.0025 part by mass of BMOV that was a vanadium compound as the component (C) and 0.1 part by mass of HQ as the phenol type compound (D), and a homogeneous solution thereof (composition A) was obtained. To another 100 parts by mass of the solution of D26E/3G (50 wt%/50 wt%), there was added 1 part by mass of PhBNa that was an aryl borate compound as the component (A), and a homogeneous solution thereof (composition B) was obtained. The two solutions were mixed together at a mass ratio of 1:1 so as to become homogeneous, and were evaluated for curing time (initially), curing property and stickiness on the surface. Further, the compositions A and B were stored in an incubator maintained at 37°C for one month and were, thereafter, measured for curing time (after stored) . The results were as shown in Table 1.

### <Examples 2 to 24 and Comparative Examples 1 to 6>

Solutions of D26E/3G (50 w%/50 wt%) containing chemical polymerization catalysts shown in Tables 1, 2 and 3 were prepared, and were cured according to the method of Example 1 to evaluate their properties. The results were as shown in Tables 1, 2 and 3.

**Table 1**

| | Composition A | | | | | | Composition B | | | |
|---|---|---|---|---|---|---|---|---|---|---|
| | Acid compound (B) | | Vanadium compound (C) | | Phenol type compound (D) | | Aryl borate compound (A) | | Other additives | |
| | | * | | * | | * | | * | | * |
| Ex. 1 | PM | 5 (16.7) | BMOV | 0.0025 (0.00788) | HQ | 0.1 (0.908) | PhBNa | 1 (2.92) | - | - |
| Ex. 2 | PM | 5 (16.7) | BMOV | 0.0025 (0.00788) | HQ | 0.1 (0.908) | PhBNa | 0.4 (1.17) | - | - |
| Ex. 3 | PM | 5 (16.7) | BMOV | 0.0025 (0.00788) | HQ | 0.1 (0.908) | PhBNa | 3 (8.77) | - | - |
| Ex. 4 | PM | 5 (16.7) | BMOV | 0.0025 (0.00788) | HQ | 0.1 (0.908) | PhBTEOA | 1 (2.13) | - | - |
| Ex. 5 | PM | 5 (16.7) | BMOV | 0.0025 (0.00788) | HQ | 0.1 (0.908) | PhBDMPT | 1 (2.19) | - | - |
| Ex. 6 | PM | 5 (16.7) | BMOV | 0.0025 (0.00788) | HQ | 0.1 (0.908) | PhBDMBE | 1 (1.95) | - | - |
| Ex. 7 | PM | 5 (16.7) | BMOV | 0.0025 (0.00788) | HQ | 0.1 (0.908) | FPhBNa | 1 (2.41) | - | - |
| Ex. 8 | PM | 5 (16.7) | BMOV | 0.0025 (0.00788) | HQ | 0.1 (0.908) | PhBTEA | 1 (2.37) | - | - |
| Ex. 9 | PM | 5 (16.7) | BMOV | 0.0025 (0.00788) | HQ | 0.1 (0.908) | PhBDMEM | 1 (2.09) | - | - |
| Ex. 10 | PM | 5 (16.7) | BMOV | 0.0025 (0.00788) | HQ | 0.1 (0.908) | BFPhBNa | 1 (2.66) | - | - |
| Ex. 11 | ** | 3 (30.6) | BMOV | 0.0025 (0.00788) | HQ | 0.1 (0.908) | PhBNa | 1 (2.92) | - | - |
| Ex. 12 | MMPS | 5 22.6 | BMOV | 0.0025 (0.00788) | HQ | 0.1 (0.908) | PhBNa | 1 (2.92) | - | - |

| | | | | | Curing time/sec. | | | | | |
|---|---|---|---|---|---|---|---|---|---|---|
| | | | | | Initially | | After stored | | Curing property | |
| Ex. 1 | | | | | 180 | | 185 | | ⊚ | |
| Ex. 2 | | | | | 210 | | 220 | | ⊚ | |
| Ex. 3 | | | | | 160 | | 165 | | ⊚ | |
| Ex. 4 | | | | | 150 | | 160 | | ⊚ | |
| Ex. 5 | | | | | 190 | | 200 | | ⊚ | |
| Ex. 6 | | | | | 170 | | 175 | | ⊚ | |
| Ex. 7 | | | | | 180 | | 185 | | ⊚ | |
| Ex. 8 | | | | | 190 | | 200 | | ⊚ | |
| Ex. 9 | | | | | 190 | | 195 | | ⊚ | |
| Ex. 10 | | | | | 160 | | 170 | | ⊚ | |
| Ex. 11 | | | | | 180 | | 185 | | O | |
| Ex. 12 | | | | | 160 | | 170 | | ⊚ | |

| | | | | | | | | | | |
|---|---|---|---|---|---|---|---|---|---|---|
| *: mass pts. (mmols) **: phosphoric acid | | | | | | | | | | |

**Table 2**

| | Composition A | | | | | | Composition B | | | |
|---|---|---|---|---|---|---|---|---|---|---|
| | Acid compound (B) | | Vanadium compound (C) | | Phenol type compound (D) | | Aryl borate compound (A) | | Other additives | |
| | | * | | * | | * | | * | | * |
| Ex. 13 | PM | 1 (3.34) | BMOV | 0.0025 (0.00788) | HQ | 0.1 (0.908) | PhBNa | 1 (2.92) | - | - |
| Ex. 14 | PM | 5 (16.7) | VOAA | 0.0025 (0.00943) | HQ | 0.1 (0.908) | PhBNa | 1 (2.92) | - | - |
| Ex. 15 | PM | 5 (16.7) | V2O5 | 0.0025 (0.0137) | HQ | 0.1 (0.908) | PhBNa | 1 (2.92) | - | - |
| Ex. 16 | PM | 5 (16.7) | BMOV | 0.005 (0.0158) | HQ | 0.1 (0.908) | PhBNa | 1 (2.92) | - | - |
| Ex. 17 | PM | 5 (16.7) | BMOV | 0.0075 (0.0236) | HQ | 0.1 (0.908) | PhBNa | 1 (2.92) | - | - |
| Ex. 18 | PM | 5 (16.7) | BMOV | 0.0025 (0.00788) | HQME | 0.1 (0.806) | PhBNa | 1 (2.92) | - | - |
| Ex. 19 | PM | 5 (16.7) | BMOV | 0.0025 (0.00788) | Ph | 0.1 (1.06) | PhBNa | 1 (2.92) | - | - |
| Ex. 20 | PM | 5 (16.7) | BMOV | 0.0025 (0.00788) | HQ | 0.02 (0.182) | PhBNa | 1 (2.92) | - | - |
| Ex. 21 | PM | 5 (16.7) | BMOV | 0.0025 (0.00788) | HQ | 0.002 (0.0182) | PhBNa | 1 (2.92) | - | |
| Ex. 22 | PM | 5 (16.7) | BMOV | 0.0025 (0.00788) | HQ | 0.05 (0.454) | PhBNa | 1 (2.92) | - | - |
| Ex. 23 | PM | 5 (16.7) | BMOV | 0.0025 (0.00788) | HQ | 0.1 (0.908) | PhBNa | 1 (2.92) | percumyl H | 2 (13.1) |
| Ex. 24 | PM | 5 (16.7) | BMOV | 0.0025 (0.00788) | HQ | 0.1 (0.908) | PhBNa | 1 (2.92) | perocta H | 2 (13.7) |

| Curing time/sec. | | | | | | | | | | |
|---|---|---|---|---|---|---|---|---|---|---|
| | Initially | | | After stored | | | | Curing property | | |
| Ex. 13 | 220 | | | 230 | | | | ⊚ | | |
| Ex. 14 | 190 | | | 195 | | | | ⊚ | | |
| Ex. 15 | 190 | | | 200 | | | | ⊚ | | |
| Ex. 16 | 150 | | | 160 | | | | ⊚ | | |
| Ex. 17 | 140 | | | 170 | | | | ⊚ | | |
| Ex. 18 | 220 | | | 225 | | | | ⊚ | | |
| Ex. 19 | 220 | | | 225 | | | | ⊚ | | |
| Ex. 20 | 230 | | | 235 | | | | ⊚ | | |
| Ex. 21 | 260 | | | 270 | | | | ⊚ | | |
| Ex. 22 | 200 | | | 210 | | | | ⊚ | | |
| Ex. 23 | 160 | | | 165 | | | | ⊚ | | |
| Ex. 24 | 160 | | | 170 | | | | ⊚ | | |

| | | | | | | | | | | |
|---|---|---|---|---|---|---|---|---|---|---|
| *: mass pts. (mmols) | | | | | | | | | | |

**Table 3**

| | Composition A | | | | | | Composition B | | | |
|---|---|---|---|---|---|---|---|---|---|---|
| | Acid compound (B) | | Vanadium compound (C) | | Phenol type compound (D) | | Aryl borate compound (A) | | Other additives | |
| | | * | | * | | * | | * | | * |
| Comp. Ex. 1 | PM | 5 (16.7) | BMOV | 0.0025 (0.00788) | - | - | PhBNa | 1 (2.92) | - | - |
| Comp. Ex. 2 | PM | 5 (16.7) . | BMOV | 0.0025 (0.00788) | BHT | 0.1 (0.454) | PhBNa | 1 (2.92) | - | - |
| Comp. Ex. 3 | PM | 5 (16.7) | BMOV | 0.0125 (0.0394) | BHT | 0.1 (0.454) | PhBNa | 1 (2.92) | - | - |
| Comp. Ex. 4 | PM | 5 (16.7) | BMOV | 0.0025 (0.00788) | HQ | 0.1 (0.908) | | - | - | - |
| Comp. Ex. 5 | - | | BMOV | 0.0025 (0.00788) | HQ | 0.1 (0.908) | PhBNa | 1 (2.92) | - | - |
| Comp. Ex. 6 | PM | 5 (16.7) | | - | HQ | 0.1 (0.908) | PhBNa | 1 (2.92) | - | - |

| | | | | | Curing time/sec. | | | | Curing property | |
|---|---|---|---|---|---|---|---|---|---|---|
| | | | | | Initially | | After stored | | | |
| Comp. Ex. 1 | | | | | 300 | | 305 | | ⊚ | |
| Comp. Ex. 2 | | | | | 330 | | 340 | | ⊚ | |
| Comp. Ex. 3 | | | | | 180 | | gelled | | ⊚ | |
| Comp. Ex. 4 | | | | | not cured | | | | | |
| Comp. Ex. 5 | | | | | not cured | | | | | |
| Comp. Ex. 6 | | | | | >3600 | | >3600 | | × | |

| | | | | | | | | | | |
|---|---|---|---|---|---|---|---|---|---|---|
| *: mass pts. (mmols) | | | | | | | | | | |

In Examples 1 to 24, the curable compositions blended with chemical polymerization catalysts of the invention were evaluated for their curing rates and curing properties. As will be obvious from the above Tables 1 and 2, favorable curing properties were exhibited in all of the Examples using the chemical polymerization catalysts of the invention.

In Comparative Example 1, on the other hand, there was added no phenol type compound (D) that was essential for the chemical polymerization catalyst of the invention, and the curing time was longer than that of the case of when the component (D) was added (Example 1) . In Comparative Example 2, a BHT (2,6-di-t-butylhydroxytoluene) that has been known as a polymerization inhibitor was added in place of the phenol type compound of the general formula (1) used in the present invention, and the curing time was longer than that of the case of when the phenol type compound of the formula (1) was added (Example 1) . In Comparative Example 3, there was used no phenol type compound of the formula (1) that was used in the invention, but the vanadium compound (component C) was used in an amount 5 times as large as that in Example 1 together with the BHT. In this case, the initial curing time was 180 seconds, and the polymerizing activity had been very improved to be comparable to that of the present invention. After stored at 37°C for one month, however, the composition itself had been gelled, and the storage stability had been greatly decreased.

Comparative Examples 4 to 6 have lacked any one of the components that were essential for the chemical polymerization catalysts. In Comparative Examples 4 and 5 that lacked aryl borate compound or the acid compound, the compositions were not at all cured. In Comparative Example 6 that lacked the vanadium compound of a valency of +IV or +V, the composition was only partly gelled even after one hour has passed, and was cured very poorly.

Next, the dental curable compositions containing chemical polymerization catalyst compositions were evaluated for their properties.

### <Example 25>

There was prepared a dental curable composition A (adhesive A for directly restoring the tooth) comprising a first solution and a second solution shown in Table 4. By using the above adhesive, the strength of adhesion was measured based on the method of the case of using the above photo-curable type composite resin (constituent components in Table 4 were by parts by mass) . As a result (Table 5), the strength of adhesion was 23.1 MPa to the enamel and was 23.3 MPa to the dentin [parentheses are standard deviations].

### <Examples 26 to 37 and Comparative Examples 7 to 12>

There were prepared adhesives B to M for directly restoring the tooth comprising first solutions and second solutions shown in Table 4. Just before the use, the adhesives were mixed together at ratios shown in Table 4 to measure their strengths of adhesion (constituent components in Table 4 were by parts by mass) . Kinds of the used composite resins and the measured strengths of adhesion were as shown in Table 5.

**Table 4**

| | First solution composition/mass pts. (mmols) | | | | | | | |
|---|---|---|---|---|---|---|---|---|
| | Acid compound (B) | | Polymerizable monomer | | Vanadium compound (C) | | Phenol type compound (D) | |
| A | PM | 20(66.8) | D2.6E | 15 | BMOV | 0.0025 | HQ | 0.1 |
| | MAC-10 | 5 (15.2) | TMPT | 10 | | (0.00788) | | (0.908) |
| B | PM | 20(66.8) | D2.6E | 15 | VOAA | 0.0025 | HQ | 0.1 |
| | MAC-10 | 5(15.2) | TMPT | 10 | | (0.0189) | | (0.908) |
| C | PM | 20(66.8) | D2.6E | 15 | BMOV | 0.005 | HQ | 0.1 |
| | MAC-10 | 5(15.2) | TMPT | 10 | | (0.0158) | | (0.908) |
| D | PM | 20(66.8) | D2.6E | 15 | BMOV. | 0.01 | HQ | 0.1 |
| | 4-META | 5(17.1) | TMPT | 10 | | (0.0315) | | (0.908) |
| E | PM | 20(66.8) | D2.6E | 15 | BMOV | 0.0025 | HQ | 0.02 |
| | MAC-10 | 5 (15.2) | TMPT | 10 | | (0.00788) | | (0.182) |
| F | PM | 20 (66.8) | D2.6E | 15 | BMOV | 0.0025 | HQ | 0.05 |
| | MAC-10 | 5 (15.2) | TMPT | 10 | | (0.00788) | | (0.454) |
| G | PM | 20(66.8) | D2.6E | 15 | BMOV | 0.0025 | HQ | 0.1 |
| | MAC-10 | 5(15.2) | TMPT | 10 | | (0.00788) | | (0.908) |
| H | PM | 20(66.8) | D2.6E | 15 | BMOV | 0.005 | HQ | 0.05 |
| | MAC-10 | 5(15.2) | TMPT | 10 | | (0.0158) | | (0.454) |
| I | PM | 20 (66.8) | D2.6E | 15 | BMOV | 0.0025 | HQ | 0.1 |
| | MAC-10 | 5(15.2) | TMPT | 10 | | (0.00788) | | (0.908) |
| J | PM | 20 (66.8) | D2.6E | 15 | BMOV | 0.0025 | - | |
| | MAC-10 | 5(15.2) | TMPT | 10 | | (0.00788) | | |
| K | PM | 20(66.8) | D2.6E | 15 | BMOV | 0.0025 | HQ | 0.1 |
| | MAC-10 | 5(15.2) | TMPT | 10 | | (0.00788) | | (0.908) |
| L | - | | D2.6E | 35 | BMOV | 0.0025 | HQ | 0.1 |
| | | | TMPT | 15 | | (0.00788) | | (0.908) |
| M | PM | 20 (66.8) | D2.6E | 15 | - | | HQ | 0.1 |
| | MAC-10 | 5(15.2) | TMPT | 10 | | | | (0.908) |

| Second solution composition/mass pts. (mmols) | | | | | | | | |
|---|---|---|---|---|---|---|---|---|
| | Polymerizable monomer | | Aryl borate compound (A) | | Phenol type compound (D) | | Other components | |
| A | HEMA | 30 | PhBTEOA | 2 | - | | water | 5 |
| | MMA | 8 | | (4.26) | | | F1 | 5 |
| | | 30 8 | PhBTEOA | | - | | water | 5 |
| | MMA | | | 2 (4.26) | | | F1 | 5 |
| C | HEMA MMA | 30 8 | PhBTEOA | | - | | water | 5 |
| | | | | 2 (4.26) | | | F1 | 5 |
| D | HEMA MMA | 30 8 | PhBTEOA | | - | | water | 5 |
| | | | | 2 (4.26) | | | F1 | 5 |
| E | HEMA | 30 | PhBTEOA | 2 | - | | water | 5 |
| | MMA | 8 | | (4.26) | | | F1 | 5 |
| F | HEMA | 30 | PhBTEOA | 2 | HQ | 0.05 | water | 5 |
| | MMA | 8 | | (4.26) | | (0.454) | F1 | 5 |
| G | HEMA | 30 | PhBTEOA | 0.5 | - | | water | 5 |
| | MMA | 9.5 | | (1.07) | | | F1 | 5 |
| H | HEMA | 30 | PhBTEOA | 5 | HQ | 0.05 | water | 5 |
| | MMA | 5 | | (10.7) | | (0.454) | F1 | 5 |
| I | HEMA | 30 | PhBNa | 2 | - | | water | 5 |
| | MMA | 8 | | (5.84) | | | F1 | 5 |
| J | HEMA | 30 | PhBTEOA | 2 | - | | water | 5 |
| | MMA | 8 | | (4.26) | | | F1 | 5 |
| K | HEMA | 30 | - | | - | | water | 5 |
| | MMA | 10 | | | | | F1 | 5 |
| L | HEMA | 30 | PhBTEOA | 2 | - | | water | 5 |
| | MMA | 8 | | (4.26) | | | F1 | 5 |
| M | HEMA | 30 | PhBTEOA | 2 | - | | water | 5 |
| | MMA | 8 | | (4.26) | | | F1 | 5 |

**Table 5**

| | Adhesive for direct restoration | Directly filled restorative | Strength of adhesion/MPa (S.D.) | |
|---|---|---|---|---|
| | | | Enamel | Dentin |
| Ex. 25 | A | photo-curable | 23.1 (4.3) | 23.3 (3.8) |
| Ex. 26 | B | photo-curable | 23.2 (3.9) | 22.8 (3.2) |
| Ex. 27 | C | photo-curable | 23.8 (3.4) | 23.1 (2.9) |
| Ex. 28 | D | photo-curable | 24.2. (3.8) | 24.1 (4.3) |
| Ex. 29 | E | photo-curable | 22.2 (2.1) | 21.9 (3.3) |
| Ex. 30 | F | photo-curable | 24.1 (3.3) | 23.6 (3.9) |
| Ex. 31 | G | photo-curable | 23.1 (3.5) | 22.9 (3.1) |
| Ex. 32 | H | photo-curable | 24.5 (3.1) | 24.2 (3.4) |
| Ex. 33 | I | photo-curable | 23.1 (2.3) | 23.3 (3.1) |
| Ex. 34 | A | chemically curable | 22.2 (2.7) | 21.5 (3.1) |
| Ex. 35 | B | chemically curable | 21.9 (2.8) | 21.1 (3.2) |
| Ex. 36 | E | chemically curable | 21.3 (3.0) | 20.7 (2.2) |
| Ex. 37 | G | chemically curable | 22.3 (3.2) | 21.0 (2.7) |
| Comp. Ex. 7 | J | photo-curable | 19.7 (3.2) | 19.9(4.2) |
| Comp. Ex. 8 | K | photo-curable | 0 | 0 |
| Comp. Ex. 9 | L | photo-curable | 0 | 0 |
| Comp. Ex. 10 | M | photo-curable | 1.9 (0.3) | 0 |
| Comp. Ex. 11 | J | chemically curable | 19.2 | (4.1) 18.1(3.4) |
| Comp. Ex. 12 | M | chemically curable | 1.4 (0.2) | 0 |

As will be obvious from Table 5 above, the adhesives for directly restoring the tooth containing the chemical polymerization catalysts exhibited large strengths of adhesion to both the enamel and the dentin even without any pre-treatment.

When the phenol type compound (D) which was essential for the chemical polymerization catalyst was not added (Comparative Examples 7 and 11), on the other hand, the strengths of adhesion were smaller than those of when the phenol type compound (D) was added (Examples 25 and 34) . Further, the adhesives which did not contain any one of the aryl borate compound, acid compound or vanadium compound exhibited very small strengths of adhesion.

### <Example 38>

There was prepared a dental curable composition (adhesive for indirectly restoring the tooth) CR-1 comprising a first past and a second past of compositions shown in Table 6.

On the other hand, the pre-treating material of the following composition was prepared being divided into a first solution and a second solution, which were mixed together just before the use each in an equal mass to treat the tooth surface. Parentheses represent parts by mass.

| | |
|---|---|
| First solution: | PM (15) |
| | MAC-10 (5) |
| | Bis-GMA (5) |
| | Acetone (10) |
| | Isopropyl alcohol (6) |
| Second solution: | Water (38) |
| | Acetone (19) |
| | Sodium p-toluenesulfinate (2) |

Next, the first paste and the second paste constituting the CR-1 were mixed together just before the use each in an equal mass to measure the strength of adhesion according to the above method of measuring the strength of adhesion of the adhesive for indirect restoration. The results were as shown in Table 7.

### <Examples 39 to 47>

There were prepared adhesives CR-2 to CR-20 for indirect restoration comprising the first pastes and the second pastes of compositions shown in Table 6. The strengths of adhesion were measured by the same method as that of Example 38 but using the above adhesives. The results were as shown in Table 7.

**Table 6**

| First paste composition/mass pts. (mmols) | | | | | | | | | | |
|---|---|---|---|---|---|---|---|---|---|---|
| | Acid compound (B) | | Polymerizable monomer | | Vanadium compound (C) | | Phenol type compound (D) | | Inorganic filler | |
| CR-1 | PM | 8(26.7) | BisGMA | 15 | BMOV | 0.0025 | HQ | 0.1 | 3Si-Zr | 50 |
| | MAC-10 | 2(6.1) | 3G | 10 | | (0.00788) | | (0.908) | 0.3Si-Ti | 50 |
| CR-2 | PM | 8(26.7) | BisGMA | 15 | BMOV | 0.0025 | HQ | 0.1 | 3Si-Zr | 50 |
| | MAC-10 | 2(6.1) | NPG | 9 | | (0.00788) | | (0.908) | 0.3Si-Ti | 50 |
| | | | MTU-6 | 1 | | | | | | |
| CR-3 | PM | 8(26.7) | BisGMA | 15 | VOAA | 0.0025 | HQ | 0.1 | 3Si-Zr | 50 |
| | MAC-10 | 2(6.1) | 3G | 10 | | (0.00943) | | (0.908) | 0.3Si-Ti | 50 |
| CR-4 | PM | 8(26.7) | BisGMA | 15 | V205 | 0.0025 | HQ | 0.1 | 3Si-Zr | 50 |
| | MAC-10 | 2(6.1) | 3G | 10 | | (0.0137) | | (0.908) | 0.3Si-Ti | 50 |
| CR-5 | PM | 8(26.7) | BisGMA | 15 | BMOV | 0.0025 | HQME | 0.1 | 3Si-Zr | 50 |
| | MAC-10 | 2(6.1) | 3G | 10 | | (0.00788) | | (0.806) | 0.3Si-Ti | 50 |
| CR-6 | PM | 8(26.7) | BisGMA | 15 | BMOV | 0.0025 | Ph | 0.1 | 3Si-Zr | 50 |
| | MAC-10 | 2(6.1) | 3G | 10 | | (0.00788) | | (1.06) | 0.3Si-Ti | 50 |
| CR-7 | PM | 8(26.7) | BisGMA | 15 | BMOV | 0.0025 | HQ | 0.1 | 3Si-Zr | 50 |
| | MAC-10 | 2(6.1) | 3G | 10 | | (0.00788) | | (0.908) | 0.3Si-Ti | 50 |
| CR-8 | PM | 8(26.7) | BisGMA | 15 | BMOV | 0.0025 | HQ | 0.05 | 3Si-Zr | 50 |
| | MAC-10 | 2(6.1) | 3G | 10 | | (0.00788) | | (0.454) | 0.3Si-Ti | 50 |
| CR-9 | PM | 8(26.7) | BisGMA | 15 | BMOV | 0.0025 | HQ | 0.1 | 3Si-Zr | 50 |
| | MAC-10 | 2(6.1) | 3G | 10 | | (0.00788) | | (0.908) | 0.3Si-Ti | 50 |
| CR-10 | PM | 8(26.7) | BisGMA | 15 | BMOV | 0.0025 | HQ | 0.05 | 3Si-Zr | 50 |
| | MAC-10 | 2(6.1) | 3G | 10 | | (0.00788) | | (0.454) | 0.3Si-Ti | 50 |

| Second paste composition/mass pts. (mmols) | | | | | | | | | | |
|---|---|---|---|---|---|---|---|---|---|---|
| | Polymerizable monomer | | Aryl borate compound (A) | | Inorganic filler | | Phenol type compound (D) | | Other components | |
| CR-1 | BisGMA | 21 | PhBNa | 2 (5.84) | 3Si-Zr | 50 | - | | - | |
| | 3G | 14 | | | 0.3Si-Ti | 50 | | | | |
| CR-2 | BisGMA | 21 | PhBTEOA | 2 (4.26) | 3Si-Zr | 40 | - | | - | |
| | HEMA | 14 | | | 0.3Si-Ti | 50 | | | | |
| | | | | | FASG | 10 | | | | |
| CR-3 | BisGMA | 21 | PhBNa | 2 (5.84) | 3Si-Zr | 50 | - | | - | |
| | 3G | 14 | | | 0.3Si-Ti | 50 | | | | |
| CR-4 | BisGMA | 21 | PhBNa | 2 (5.84) | 3Si-Zr | 50 | - | | - | |
| | 3G | 14 | | | 0.3Si-Ti | 50 | | | | |
| CR-5 | BisGMA | 21 | PhBTEOA | 2 (4.26) | 3Si-Zr | 50 | - | | - | |
| | 3G | 14 | | | 0.3Si-Ti | 50 | | | | |
| CR-6 | BisGMA | 21 | PhBTEOA | 2 (4.26) | 3Si-Zr | 50 | - | | - | |
| | 3G | 14 | | | 0.3Si-Ti | 50 | | | | |
| CR-7 | BisGMA | 21 | FPhBNa | 2 (4.83) | 3Si-Zr | 50 | - | | - | |
| | 3G | 14 | | | 0.3Si-Ti | 50 | | | | |
| CR-8 | BisGMA | 12 | PhBTEOA | 2 (4.26) | 3Si-Zr | 50 | HQ | 0.05 (0.454) | - | |
| | 3G | 18 | | | 0.3Si-Ti | 50 | | | | |
| CR-9 | BisGMA | 12 | PhBTEOA | 2 (4.26) | 3Si-Zr | 50 | - | | percuryl H | 1 (6.57) |
| | 3G | 18 | | | 0.3Si-Ti | 50 | | | | |
| CR-10 | BisGMA | 12 | PhBTEOA | 2 (4.26) | 3Si-Zr | 50 | HQ | 0.05 (0.454) | perocta H | 1 (6.84) |
| | 3G | 18 | | | 0.3Si-Ti | 50 | | | | |

**Table 7**

| | Adhesive for indirect restoration | Strength of adhesion/MPa (S.D.) | |
|---|---|---|---|
| | | Enamel | Dentin |
| Ex. 38 | CR-1 | 23.7 (4.3) | 23.3 (3.3) |
| Ex. 39 | CR-2 | 23.5 (2.9) | 23.8 (2.8) |
| Ex. 40 | CR-3 | 23.7 (3.4) | 23.1 (2.4) |
| Ex. 41 | CR-4 | 23.5 (3.7) | 23.3 (3.2) |
| Ex. 42 | CR-5 | 22.6 (3.2) | 22.2 (2.4) |
| Ex. 43 | CR-6 | 22.6 (3.9) | 22.3 (2.3) |
| Ex. 44 | CR-7 | 23.4 (2.5) | 23.2 (3.1) |
| Ex. 45 | CR-8 | 24.2 (4.2) | 23.9 (4.6) |
| Ex. 46 | CR-9 | 25.2 (4.4) | 24.7 (4.2) |
| Ex. 47 | CR-10 | 25.0 (3.9) | 24.9 (3.8) |

As will be obvious from Table 7 above, the adhesives for indirectly restoring the tooth containing the chemical polymerization catalysts of the invention exhibited large strengths of adhesion to both the enamel and the dentin.

## Claims

1. Use of a phenol type compound represented by the following general formula (1), 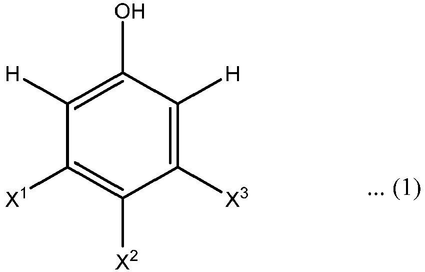
wherein X¹, X² and X³ are each a hydrogen atom or a substituent selected from the group consisting of a hydroxyl group, an alkoxy group and an alkyl group;
for improving the polymerizing activity of a dental chemical polymerization catalyst, wherein the use is not in a method of treatment of the human or animal body by surgery or therapy, and wherein the dental chemical polymerization catalyst comprises:
(A) an aryl borate compound;
(B) an acid compound which is an acid group-containing polymerizable monomer;
(C) a vanadium compound having a valency of +IV or +V; and
(D) the phenol type compound represented by general formula (1);
said acid compound (B) being contained in an amount of 0.1 to 100 mols per mole of the aryl borate compound (A) and said vanadium compound (C) being contained in an amount of 0.0005 to 0.015 mol per mol of said aryl borate compound (A), and wherein said phenol type compound (D) is contained in an amount of 0.05 to 200 mols per mol of said vanadium compound (C) and said phenol type compound (D) is not the compound in which X¹ and X³ are hydrogen atoms and X² is a methoxy group.

2. The use according to claim 1, wherein said chemical polymerization catalyst further contains an organic peroxide (E).

3. The use according to claim 1, wherein a compound containing not less than two phenolic hydroxyl groups is used as said phenol type compound (D).

4. The use according to claim 3, wherein a hydroquinone is used as said phenol type compound (D).

5. The use according to claim 1, wherein said aryl borate compound (A) is stored in a form of package separate from said acid compound (B), vanadium compound (C) and phenol type compound (D), and the components (A) to (D) are all mixed together at the time of use of the dental chemical polymerization catalyst.

## Patentansprüche

1. Verwendung einer Verbindung vom Phenoltyp, dargestellt durch die folgende allgemeine Formel (1), 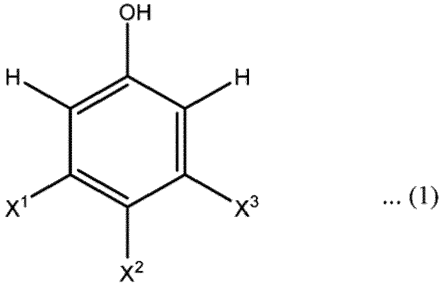
wobei X¹, X² und X³ jeweils ein Wasserstoffatom oder ein Substituent, der aus der Gruppe, bestehend aus einer Hydroxylgruppe, einer Alkoxygruppe und einer Alkylgruppe, ausgewählt ist, sind;
zur Verbesserung der Polymerisationsaktivität eines zahnmedizinischen chemischen Polymerisationskatalysators, wobei die Verwendung nicht in einem Verfahren zur Behandlung des menschlichen oder tierischen Körpers durch Chirurgie oder Therapie erfolgt, und wobei der zahnmedizinische chemische Polymerisationskatalysator Folgendes umfasst:
(A) eine Arylboratverbindung;
(B) eine Säureverbindung, bei der es sich um ein säuregruppenhaltiges polymerisierbares Monomer handelt;
(C) eine Vanadiumverbindung mit einer Wertigkeit von +IV oder +V, und
(D) die Verbindung vom Phenoltyp, dargestellt durch die allgemeine Formel (1);
wobei die Säureverbindung (B) in einer Menge von 0,1 bis 100 Mol pro Mol der Arylboratverbindung (A) enthalten ist und die Vanadiumverbindung (C) in einer Menge von 0,0005 bis 0,015 Mol pro Mol der Arylboratverbindung (A) enthalten ist, und wobei die Verbindung vom Phenoltyp (D) in einer Menge von 0,05 bis 200 Mol pro Mol der Vanadiumverbindung (C) enthalten ist und die Verbindung vom Phenoltyp (D) nicht die Verbindung ist, in der X¹ und X³ Wasserstoffatome sind und X² eine Methoxygruppe ist.

2. Verwendung nach Anspruch 1, wobei der chemische Polymerisationskatalysator ferner ein organisches Peroxid (E) enthält.

3. Verwendung nach Anspruch 1, wobei eine Verbindung, die nicht weniger als zwei phenolische Hydroxylgruppen enthält, als die Verbindung vom Phenoltyp (D) verwendet wird.

4. Verwendung nach Anspruch 3, wobei ein Hydrochinon als Verbindung vom Phenoltyp (D) verwendet wird.

5. Verwendung nach Anspruch 1, wobei die Arylboratverbindung (A) in einer Verpackungsform getrennt von der Säureverbindung (B), der Vanadiumverbindung (C) und der Verbindung vom Phenoltyp (D) gelagert wird und die Komponenten (A) bis (D) zum Zeitpunkt der Verwendung des zahnmedizinischen chemischen Polymerisationskatalysators alle zusammen gemischt werden.

## Revendications

1. Utilisation d'un composé de type phénol représenté par la formule générale suivante 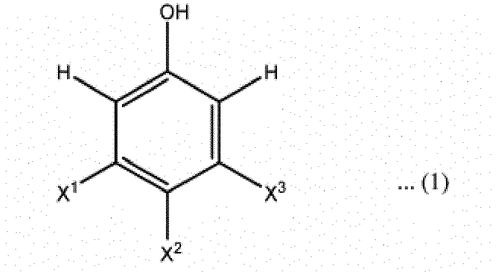
dans laquelle X¹, X² et X³ sont chacun un atome d'hydrogène ou un substituant choisi dans le groupe constitué par un groupe hydroxyle, un groupe alcoxy et un groupe alkyle ;
pour améliorer l'activité de polymérisation d'un catalyseur de polymérisation chimique dentaire, dans laquelle l'utilisation n'est pas dans un procédé de traitement du corps humain ou animal par chirurgie ou thérapie, et dans laquelle le catalyseur de polymérisation chimique dentaire comprend :
(A) un composé de borate d'aryle ;
(B) un composé acide qui est un monomère polymérisable contenant un groupe acide ;
(C) un composé de vanadium ayant une valence de +IV ou +V; et
(D) le composé de type phénol représenté par la formule générale (1) ;
ledit composé acide (B) étant contenu en une quantité de 0,1 à 100 moles par mole du composé de borate d'aryle (A) et ledit composé de vanadium (C) étant contenu en une quantité de 0,0005 à 0,015 mole par mole dudit composé de borate d'aryle (A), et dans laquelle ledit composé de type phénol (D) est contenu en une quantité de 0,05 à 200 moles par mole dudit composé de vanadium (C) et ledit composé de type phénol (D) n'est pas le composé dans lequel X¹ et X³ sont des atomes d'hydrogène et X² est un groupe méthoxy.

2. Utilisation selon la revendication 1, dans laquelle ledit catalyseur de polymérisation chimique contient en outre un peroxyde organique (E).

3. Utilisation selon la revendication 1, dans laquelle un composé ne contenant pas moins de deux groupes hydroxyle phénoliques est utilisé en tant que ledit composé de type phénol (D).

4. Utilisation selon la revendication 3, dans laquelle une hydroquinone est utilisée comme ledit composé de type phénol (D).

5. Utilisation selon la revendication 1, dans laquelle ledit composé de borate d'aryle (A) est stocké sous une forme de paquet séparé dudit composé acide (B), du composé de vanadium (C) et du composé de type phénol (D), et des composants (A) à (D) sont tous mélangés ensemble au moment de l'utilisation du catalyseur de polymérisation chimique dentaire.
